(19) Europäisches Patentamt
European Patent Office
Office européen des brevets

(11) Publication number: **0 123 446**
**B1**

(12) # EUROPEAN PATENT SPECIFICATION

(45) Date of publication of patent specification: **31.05.89**

(51) Int. Cl.[4]: **C 07 D 407/04,** A 61 K 31/335

(21) Application number: **84302020.7**

(22) Date of filing: **26.03.84**

(54) **2-Furylbutyrolactone derivatives, their preparation, compositions containing them, and their use for the modulation of the immune system in mammals.**

(30) Priority: **24.03.83 US 478331**

(43) Date of publication of application:
**31.10.84 Bulletin 84/44**

(45) Publication of the grant of the patent:
**31.05.89 Bulletin 89/22**

(84) Designated Contracting States:
**AT BE CH DE FR GB IT LI LU NL SE**

(56) References cited:
**US-A-4 287 205**
**Chemical Abstracts vol. 95, no. 7, 17 August 1981, Columbus, Ohio, USA; G. FODOR et al. The search for new cancerostatic agents", page 756, column 1, abstract no. 62551a in combination with Chemical Substance Index, vol. 95, page**
**TETRAHEDRON LETTERS, no. 12, 1978, Oxford, GB; J.P. JACOBSEN et al. "Revision of the structures of the naturally occurring acyl tetronic acids: dehydrocarolic acid, terrestric acid and carlic acid", pages 1081-1083**

(73) Proprietor: **NATIONAL FOUNDATION FOR CANCER RESEARCH, INC.**
**7316, Wisconsin Avenue Suite 851**
**Washington D.C. 20014 (US)**

(72) Inventor: **Szent-Gyorgyl, Albert**
**Seven Winds Woods Hole**
**Massachusetts 02543 (US)**
Inventor: **Fodor, Gabor B.**
**829, Augusta Avenue**
**Morgantown West Virginia 26505 (US)**
Inventor: **Veltri, Robert W.**
**280a Colwyn Terrace**
**West Chester Pennsylvania 19355 (US)**

(74) Representative: **Tubby, David George et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 123 446 B1

## Description

The present invention relates generally to 2-furylbutyrolactone derivatives, to their preparation, to compositions containing them and to their use as immunomodulatory agents.

Diseases or disorders of the immune system are a serious problem in modern medical practice, causing many distressing disorders in humans and other animals; disorders of the immune system are also thought to be implicated in at least some cancers.

We have now discovered a series of 2-furylbutyrolactone derivatives (prepared by reacting a 2,5-dihydrofuran derivative with a 2,3-dihydrobutyrolactone compound, especially ascorbic acid) which have the ability to modulate the activity of the immune system.

U.S. Patent Specification No. 4,287,205 discloses a series of cyclic acetals, some of which can be prepared in a similar way and which were disclosed as hypotensive and analgesic agents. Specifically, Example 3 of said U.S. Patent Specification discloses a compound prepared by reaction of 2-methyl-2,5-dimethyl-2,5-dihydrofuran with *L*-ascorbic acid, which is similar to the preferred first step in the preparation of the compounds of the invention, but that compound is structurally dissimilar from the compounds of the invention, which are prepared and isolated in a form free or essentially free from the cyclic acetals of said U.S. Patent Specification.

The compounds of the invention have the general formula (I):

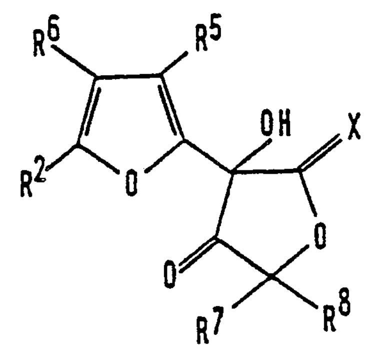

(I)

in which:

$R^2$ represents a hydrogen atom or a $C_1$—$C_6$ alkyl group;

$R^5$ and $R^6$ are the same or different and each represents a hydrogen atom or a $C_1$—$C_6$ alkyl group;

$R^7$ and $R^8$ are the same or different and each represents a hydrogen atom or a $C_1$—$C_6$ alkyl group, or $R^7$ represents a group of formula:

$$R^9(H)C(OR^{10})—$$

in which:

$R^9$ represents a group of formula

$$—(CH_2)_n—OR^{11};$$

*n* is an integer from 1 to 3; and $R^{10}$ and $R^{11}$ are the same or different and each represents a hydrogen atom, a $C_1$—$C_6$ alkyl group, a phenyl group or a hydroxy-substituted $C_1$—$C_6$ alkyl group; and

X represents an oxygen or sulphur atom or the group =NH; and

where $R^7$ contains a hydroxy group, hemiketals thereof; and

adducts of said compounds of general formula (I) or said hemiketals with an anhydride or amide of succinic acid.

These compounds may be produced by reacting (preferably approximately equal amounts of) a compound of the general formula (II):

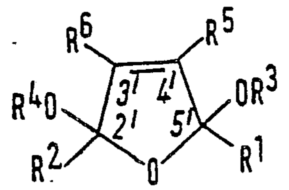

(II)

wherein: $R^1$ and $R^2$ are the same or different and each represents a hydrogen atom or a $C_1$—$C_6$ alkyl group, provided that at least one is hydrogen; $R^3$ and $R^4$ are the same or different and each represents a $C_1$—$C_6$

alkyl group or an aryl group; and $R^5$ and $R^6$ are as defined above with a compound of the general formula (III):

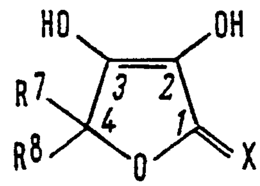

(III)

in which

X, $R^7$ and $R^8$ are as defined above;

treating the product with an anhydride or an amide of succinic acid;

separating the product as an adduct with said anhydride or amide from the reaction mixture; and

if desired, dissociating said product into said compound of general formula (I) or hemiketal thereof and said anhydride or amide.

Recovery of the pure product may be facilitated by treatment or purification of the intermediate by any of the several techniques known to one of ordinary skill in the art such as, for example, charcoal filtration, ion exchange or gel chromatography.

When $R^7$ contains a hydroxyl group in the α, β, or γ position, $R^7$ may form the hemiketal ring closure at carbon 3 of the butyrolactone with protonation of the carbonyl group on the same carbon atom, to give the hemiketal of general formula (IV):

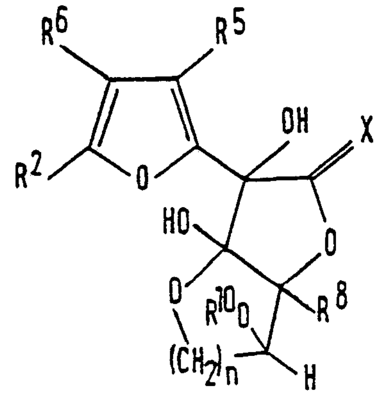

(IV)

These rings will ordinarily partially rearrange, in aqueous or highly polar media to give tautomeric equilibrium products

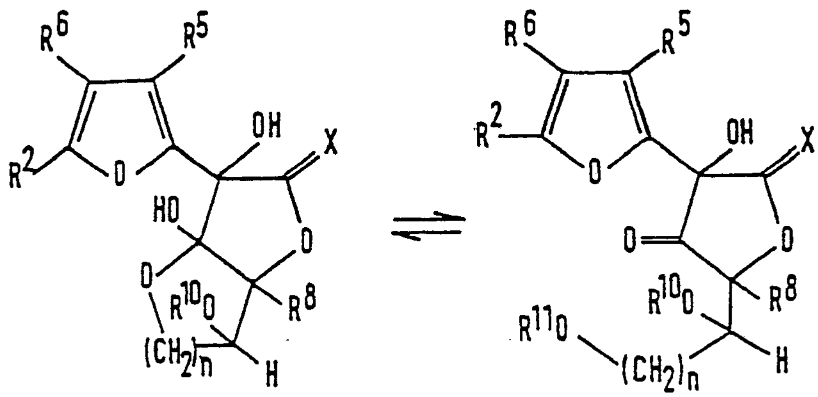

wherein $R^2$, $R^5$, $R^6$, $R^7$, $R^8$, $R^{10}$, $R^{11}$, X and *n* are as defined above.

Preferred compounds of general formula (II) are those in which:

$R^1$ represents a hydrogen atom or a $C_1$—$C_3$ alkyl group;

$R^2$ represents a $C_1$—$C_3$ alkyl group;

$R^3$ and $R^4$ are the same or different and each represents a $C_1$—$C_3$ alkyl group; and

$R^5$ and $R^6$ are the same or different and each represents a hydrogen atom or a $C_1$—$C_3$ alkyl group, more preferably in which:

$R^2$ represents a methyl group; and

$R^5$ and $R^6$ each represent hydrogen atoms.

The most preferred compound is 2-methyl-2,5-dimethoxy-2,5-dihydrofuran.

Preferred compounds of general formula (III) are those in which:

X represents an oxygen atom;
$R^8$ represents a hydrogen atom or a $C_1$—$C_3$ alkyl group; and
$R^7$ represents said group of formula:

$$R^9(H)C(OR^{10})—$$

in which:
$R^9$ represents $—(CH_2)_nOR^{11}$
*n* is an integer from 1 to 3; and
$R^{10}$ and $R^{11}$ are the same or different and each represents a hydrogen atom or a $C_1$—$C_3$ alkyl group, more preferably in which:
$R^9$ represents $—CH_2OH$; and
$R^{10}$ represents a hydrogen atom.

The most preferred compound is *L*-ascorbic acid.

The preferred compounds and more preferred compounds of general formula (I) are hence those in which $R^2$, $R^5$, $R^6$, $R^7$, $R^8$ and X are as defined above.

Where reference is made to $C_1$—$C_6$ alkyl groups, these may be straight or branched chain groups, preferably the methyl, ethyl, propyl, isopropyl, butyl, isobutyl, sec-butyl, t-butyl, pentyl, isopentyl, neopentyl, t-pentyl, hexyl or isohexyl groups, of which the $C_1$—$C_3$ groups, i.e. methyl, ethyl, propyl and isopropyl, are more preferred and methyl is most preferred.

Preferably, the molar ratio of said compound (II) to said compound (III) is from 2:1 to 1:2, more preferably 1:1.

Preferably, said anhydride or amide of succinic acid is preferably succinic anhydride, succinimide or *N*-methylsuccinimide, and preferably said anhydride or amide is employed in an amount of at least 0.5 mole per mole of said compound (I), more preferably 0.5 mole.

The compounds of the invention can be formulated with generally known pharmaceutical carriers or diluents into compositions which can be administered to animals and humans. These compositions show immunomodulatory and cytotoxic activity at very low acute toxicity values.

It is believed that the cytotoxic activity of these compositions may be attributable to stimulation of the immune system by the compounds or to the cytotoxicity of the compounds *per se* or to a combination of both effects.

Because of their cytotoxic activity, these compositions may be used in cancer therapy for a broad range of cancers. The compositions may be used in immune therapy of depressed immune systems, as in the case of AIDS (Acquired Immune Deficiency Syndrome).

The immune system is one of the primary defenses against disease-bearing microbes and other foreign protein in higher animals. An immune response is mediated by the action of specific antibody proteins which react to specific antigens. Antigens are substances of fairly high molecular weight, often proteins, which are foreign to an individual's body. They are most frequently located on the outer surfaces of cells. Potential antigens can be found on pollen grains, tissue grafts, synthetics, animal parasites, viruses, and bacteria.

In humans, many potential antigens never pass the body's first two defense lines and therefore never trigger the immune system. These two defense lines consist firstly of the skin, mucous membranes, tears, and stomach acid and secondly of specialized white blood cells (granulocytes and monocytes) and macrophages which destroy pathogens and other potential antigens by phagocytosis, that is by engulfing and destroying the foreign material. These white blood cells and macrophages are called phagocytes. When pathogens or other foreign substances do pass the body's first two defense lines, the immune response begins.

There are two principal immune defense systems, humoral and cellular, both of which react to antigens. Humoral immunity is due to circulating antibodies which are found in the gamma globulin fraction of the plasma proteins. When plasma is centrifuged at high speeds its component proteins separate by weight into sections called fractions. Antibodies are usually found in the fraction whose components have a molecular weight of approximately 156,000. This particular fraction has been named the gamma globulin fraction. Humoral immunity forms a major defense against bacterial infections. Cellular immunity is partly due to lymphocyte products called lymphokines. This type of immunity is responsible for delayed allergic reactions, rejection of transplants of foreign tissue, and rejection of tumor cells. It is the major defense against infections due to viruses, fungi, and a few bacteria such as the tubercle bacillus.

Specialized white blood cells called lymphocytes are responsible for both humoral and cellular immunity. Lymphocyte precursor cells are made in the bone marrow of adult humans followed by migration to various organs or in the yolk sac of a developing fetus followed by migration into the foetus and then to various organs. In humans, some of these precursor cells migrate to the thymus, which is a two-lobed, glandular appearing structure located in the upper chest just behind the sternum, where they are transformed into T-lymphocytes, which are involved in cellular immunity. In humans, the rest of the precursor cells migrate to the spleen where they are transformed into B-lymphocytes, which are involved in humoral immunity. The T- and B-lymphocytes are structurally indistinguishable although they function

differently and can be distinguished through various chemical means. The mature lymphocytes circulate in the blood and can also be found in the lymph nodes as well as the spleen and thymus.

Humoral immunity is mediated by the B-lymphocytes which have receptors for particular antigens on their cell surfaces. They seem to be very specific and each type of B-lymphocyte reacts to only one antigen. When bacteria or viruses, for example, invade an organism, B-lymphocytes react to and combine with the antigens on the bacterial or viral surface and the lymphocyte is stimulated to divide. Its daughter cells are transformed into specialized cells called plasma cells. These calls produce and then secrete large quantities of antibodies into the general circulation. The antibodies are specific for the antigens which stimulated their production and react only with those antigens. Antibodies known as agglutinins cause several antigen-containing substances to agglutinate or clump together. This keeps the substance from spreading to the tissues and allows the phagocytes to capture or the lymph nodes to filter the invading material. Other antibodies act by opening holes in bacterial cell walls, thereby killing the bacteria. These are known as lysins. Antibodies called antitoxins combine with toxins produced by bacteria and thereby neutralize them.

Once a pathogen invades the body and the immune response begins, antibodies can be made in several hours. This initial reaction is called the primary response or primary immunization. However, during that time, the pathogens have also been dividing and sometimes producing toxin, either of which can result in various disease symptoms. It may take days or weeks before enough antibodies are made to eliminate all the pathogens but, once they disappear, the disease symptoms disappear as well. The lymphocytes, plasma cells and antibodies remain and circulate in the blood so that, if the same pathogens enter the body a second time, the lymphocytes react immediately and start antibody production. The response of the sensitized lymphocytes is called the secondary response. The secondary response results in the production of higher levels of antibody than were produced during the primary response. So many antibodies are produced so rapidly that the microbes are unable to divide and cause disease. Humoral immunity is known as immediate hypersensitivity due to the fact that a previously exposed organism can respond within minutes to an antigen, as in the case of hay fever. Another example of immediate hypersensitivity would be anaphylactic shock, an extreme allergic reaction that sometimes occurs when an individual is exposed to an antigen to which he has been sensitized. At times, this humoral response to the antigen can result in death.

Humoral immunity can be both naturally and artificially induced. In the case of active natural immunity, an individual's lymphocytes continue to circulate and activate the production of antibodies after an infection. This active natural immunity lasts for many years or even a lifetime. An infant receives antibodies from the colostrum (milk secreted by the mother) the first few days after birth, which gives it immunity during the first year of its life. This is known as passive natural immunity since the infant is not involved in the actual production of the antibodies. Active artificial immunity is induced by injecting dead or weakened microbes into an individual. Their surface antigens can still trigger lymphocyte production of antibodies but these microbes do not cause the disease symptoms that their more virulent forms do. When the individual is later exposed to the virulent microbe, he is already sensitized and immediately responds with a massive production of antibodies. Active artificial immunity may last many years or permanently with booster shots. There is also a form of passive artificial immunity which provides protection for about one month. This temporary immunity is brought about by injecting antibodies obtained from another person or animal into an individual. It is usually only used in crisis situations and epidemics. Because the lymphocytes are bypassed, they neither make antibodies nor "remember" the antigen, which accounts for the temporary effect of this method.

In cellular immunity, in contrast to humoral immunity, circulating antibodies are not detectable. The T-lymphocytes which mediate this type of immunity are activated when they encounter antigens on cells from another individual, as in the case of transplants, tumours, or viruses. Like B-lymphocytes, T-lymphocytes are specific and each type reacts with only one antigen. The lymphocytes enlarge, divide, and produce lymphokines which participate in the attack on the foreign protein. They also stimulate the phagocytic activity of macrophages. Although immunological memory exists as with humoral immunity, the response is much slower. It may take as long as ten or twelve hours to develop a response in a previously sensitized individual and cellular immunity is therefore known as delayed hypersensitivity. The allergic reaction to poison ivy, poison oak, and poison sumach, the red splotch seen in a positive tuberculin skin test, and rejection of transplant tissue are all cellular immune responses.

Immunomodulating agents activate or inhibit the process of lymphocyte proliferation. Normal lymphocyte proliferation is due to various interactions between antigens, macrophages, T- and B-lymphocytes as well as certain chemicals. For example, the presence of a particular antigen activates a particular T- or B-lymphocyte. Additionally, certain B-lymphocytes can be activated by active T-lymphocytes while others are independent of the T-lymphocytes and are activated only by antigens. Activated T-lymphocytes can cause macrophages to produce a molecule known as interleukin 1 (IL—1) which, in turn, activates both T- and B-lymphocytes. Activated T-lymphocytes can also produce a molecule known as interleukin 2 (IL—2) which further induces T-lymphocyte activation. Chemicals, called mitogens, can trigger DNA synthesis and mitosis, which are signs of activity in T- or B-lymphocytes. Some mitogens affect only one type of lymphocyte while others affect many types. Immunomodulating agents of various kinds and in varying amounts affect the complex interactions between the components of the immune system. As will be shown, the compounds and compositions of this invention act as immune stimulators or

suppressors, depending upon the dosage, and affect both T- and B-lymphocytes.

Although the immune system is a major defense against substances which can cause disease, it cannot distinguish between helpful and harmful foreign substances and destroys both. It would be useful in many instances to have a means of regulating the immune system without harming the individual. The compounds and compositions of this invention exhibit such modulating or regulatory effects and have potential for use in the treatment of various immune disorders.

The immune system has been linked to some aspects of aging and may be important in protecting against cancer. The system is necessary for the recognition of changing or aging cells, such as worn out red blood cells, and their subsequent destruction, and for this reason is vital to normal body functions. One theory in the case of cancer is that the transformation of cells to the malignant state may occur fairly frequently but these changed cells are recognized as "not self" and destroyed. Some carcinogens may work by depressing this immune response rather than by actual transformation of cells to a malignant state. This would mean that the body would no longer destroy the naturally transformed cells and a cancerous growth could result. Immunostimulation could be useful in treating cancers of this type. When used as adjunctive therapeutic agents in chemo- or chemoimmuno-therapy, the compounds and compositions of this invention can affect the growth of tumours.

Some of the methods of treating cancer, for example surgery, chemotherapy or radiation, can result in a suppression or drastic variation of the normal functions of the immune system. Immunostimulatory drugs, such as the compounds and compositions of this invention, can be very effective in combating and/or preventing various infections which can result due to the depressed immune system. Some immunomodulating agents which are currently being studied for use in these situations are cyclosporin-A, levamisole and isoprinosine (inosiplex). The compounds and compositions of this invention will be novel therapeutic tools for use in this and other situations involving the immune system.

There are times when the individual's immunological response causes more damage or discomfort than the invading microbes or foreign material, as in the case of allergic reactions. Suppression of the immune response in these cases would be desirable.

Occasionally, the immunological mechanisms become sensitized to some part of the individual's own body, causing interference with or even destruction of that part. The ability to distinguish between "self" and "not self" is impaired and the body begins to destroy itself. Some examples of these autoimmune diseases in man are rheumatoid arthritis, certain hemolytic anaemias, rheumatic fever, thyroiditis, ulcerative colitis, myasthenia gravis, glomerulonephritis (a kidney disease), allergic encephalomyelitis, continuing nerve and liver destruction which sometimes follows viral hepatitis, and possibly multiple sclerosis. Some forms of autoimmunity come about as the result of trauma to an area usually not exposed to lymphocytes such as neural tissue or the lens of the eye. When the tissues in these areas become exposed to lymphocytes, their surface proteins can act as antigens and trigger the production of antibodies and cellular immune responses which then begin to destroy those tissues. Other autoimmune diseases develop after exposure of the individual to antigens which are antigenically similar to, that is cross-react with, the individual's own tissue. Rheumatic fever is an example of this type of disease in which the antigen of the streptococcal bacterium which causes rheumatic fever is cross-reactive with parts of the human heart. The antibodies cannot differentiate between the bacterial antigens and the heart muscle antigens and cells with either of those antigens can be destroyed. Suppression of the immune system in these autoimmune diseases could be useful in minimizing or eliminating the effects of the disease.

Circulating antibodies and cellular immune responses play a role in the rejection of transplanted tissues and organs. Unless the donor is the identical twin of the recipient or is the individual himself, the recipient's lymphocytes recognize the transplant as "not self" and immediately respond to destroy it. The exceptions to this situation are transplants to non-vascularized areas (privileged sites), such as the cornea of the eye, where lymphocytes do not circulate and therefore are not sensitized and do not prompt an immune response. It is currently difficult to suppress the immune reaction to prevent rejection of the transplant without severely damaging the patient in other ways. The patient must also be given massive doses of antibiotics because his own defenses against infection have been suppressed. The compounds and compositions of this invention could be valuable in establishing tolerance to the transplant through controlled modulation of the immune system.

Because of the immune system's vital roles in defense against disease and in normal body functions, compounds and compositions, such as those disclosed in this invention, which can enhance or diminish that role are of importance. They are particularly useful when the normal action of the system has been disturbed or has caused the destruction of harmless foreign materials. They can also assist by stimulating the system's response to harmful antigens and thus shorten the course of various diseases.

The compositions of the invention, when administered to mammalian lymphocytes, have a profound effect on their ability to reproduce, as measured by their ability to synthesize DNA. This effect is bimodal. In higher concentrations, the compositions act as immunosuppressants, while in lower concentrations they act as immunostimulants.

This immunomodulatory effect is seen *in vitro* in human blood lymphocytes and *in vivo* and *in vitro* in mouse spleen lymphocytes.

Various immunomodulatory studies have been conducted using these compositions. In these studies, T- and B-lymphocytes were isolated from human blood and mice spleens. Five strains of mice were used as

lymphocyte donors, BALB/C, C57BL/6, $BDF_1$, SJL/J and DBA/2. The lymphocytes were then treated with plant proteins called lectins which act as mitogens.

Mitogens are substances which stimulate DNA synthesis and mitosis. The mitogens used in these studies were phytohaemagglutinin (PHA), which is isolated from the red kidney bean, and concanavalin-A (Con-A), which is isolated from the jack bean. Con-A binds to specific receptors (glycoproteins) containing mannosyl or glucosyl moieties and stimulates all murine T-cells to synthesize DNA, divide, and release lymphokines. Con-A in a soluble form allows distinction between T- and B-cells in the mouse, because, although both T- and B-cells can bind $10^6$ molecules of Con-A per cell, only T-cells are stimulated when this lectin is presented in a soluble form. PHA stimulates only subpopulations ($T_2$ cells) of T- or B-cells. In the mouse, PHA activates a subpopulation of T-cells and does not stimulate B-cells. In humans, both T- and B-cells are probably stimulated. The activation of B-cells may be indirect and mediated by the release of soluble mediators from PHA-activated T-cells.

At doses ranging from 1 ng to $10^5$ ng, the compounds of this invention have profound effects on lymphocyte mitogenesis when tested directly *in vitro* versus human lymphocytes. At lower doses, 1 to $10^3$ ng, there is stimulation, above control levels, of PHA or Con-A mediated mitogenesis. At high doses, $10^4$ to $10^5$ ng, there is a profound suppression of the mitogenetic activity of lymphocytes treated with mitogens PHA and Con-A.

*In vivo* treatment of C57BL/6 mice comprising intraperitoneal (i.p.) administration of varying doses of the compounds for 4—12 days and subsequent testing of spleen lymphocytes with PHA and Con-A reveals a similar pattern to that of the *in vitro* system at doses of the compounds of 400 and 800 mg/kg versus 50 to 200 mg/kg. The observed suppression, at high doses, or stimulation, at low doses, showed statistically significant differences, with $P < 0.05$.

Other experiments have been done to test the effect of the compounds on the antibody response to specific antigens, bovine serum albumin and keyhold limpet haemocyanin. It is found that high doses of the compounds do not inhibit the secondary immune response to the antigen but instead significantly enhance this response. Such doses, which routinely suppressed the *in vivo* and *in vitro* cell mediated response to mitogens, did not significantly suppress humoral immunity. These data demonstrate the possibility of regulation of specific antibody production through immunostimulation.

In the experiments described below, the trends observed with high doses or low doses of the compound follow the general pattern noted above. However, differences can be noted in the actual response to the compounds of PHA versus Con-A stimulated lymphocytes. This may be due to the fact that Con-A and PHA do not stimulate the same kinds of lymphocytes. Differences can also be noted in the responses of the various strains of mice. These could reflect genetic variation with respect to response of lymphocytes to mitogens. Differences observed in the *in vivo* versus *in vitro* response can be due to differences in *in vivo* and *in vitro* metabolism and to the difference between the human and murine lymphocytes.

In all these experiments, it should be noted that control cultures not stimulated with mitogens or specific antigens, and which, therefore, could be considered resting cells, were not affected by the compounds. The dramatic stimulatory or inhibitory effects were only demonstrated in cells with active DNA synthesis, that is those cells undergoing cell division.

Tests were also done to determine the immunomodulatory effects of the compounds on the cytotoxic lymphocyte activity of sensitized lymphocytes obtained from tumour dormant DBA/2 mice. The experimental model involved establishment of the dormant state in DBA/2 mice and then conducting studies to assess the effect of the compounds disclosed in this invention on the tumour dormant state. The animal model described above mimics a suspected tumour dormant state that is suggested by clinical observations of human recurrent breast tumours and melanomas many years after apparent cure of the primary tumours.

A. Lymphocyte Stimulation Assay Protocols

The purpose of this assay is to challenge lymphocytes in microcultures with one or more polyclonal mitogens at concentrations that will induce mitogenesis within 72 hours of stimulation.

In the mouse model, spleens were removed from different strains of mice and the spleen cells were teased free and suspended in RPMI—1640 tissue culture medium at a concentration of usually $1 \times 10^7$ ml. These cells were dispensed at a concentration of $5.0 \times 10^5$ cells per microculture well in a 96 well flat bottom tissue culture plate. All testing of the compounds of this invention or mitogens were done in replicates of 10. The cells were then treated with PHA, Con-A, specific antigens, or buffer only. Where *in vitro* testing was done, varying concentrations of the compounds of this invention or buffer only were added to the cells as well. After treatment with the mitogens and/or compounds of this invention, the cells were incubated for 72 hours. The response of these cells to mitogens and/or the compounds of this invention was assessed using $^{14}C$-thymidine labeling on the fourth day at a concentration of 0.01 microCi/well. The labeled cells were harvested using a lymphocyte harvestor and placed onto fibrous paper discs. A scintillation cocktail was added to vials containing the discs and the results were obtained using a LKB-liquid scintillation counter (Model 1216/Rackbeta II). The data is expressed as a ratio of the cpm (counts per minute) of the mitogen stimulated group to the control (non-mitogen) treated group = lymphocyte stimulation index (LSI).

The lymphocyte stimulation assay may be performed directly on spleen cells obtained from various strains of mice not treated with the compounds of this invention or on lymphocytes obtained from human plasma. In such experiments, the compounds of this invention were added directly to the spleen lymphocytes *in vitro* and the assay was completed and results interpreted. Alternatively, the mice may be treated *in vivo* with the compounds of this invention by intraperitoneal multi-dose schedules, followed by sacrifice and harvesting spleen lymphocytes. These cells were then tested for their response to polyclonal mitogens or specific antigens used to immunize the mice. In this system however, no additional compound was added to the *in vitro* lymphocyte stimulation assay system.

B. The Secondary Response Studies

The effect of the compounds of this invention on the immune response to specific antigens involved immunization of mice with specific antigens such as bovine serum albumin (BSA) or keyhold limpet haemocyanin (KLH). Following immunization, it is possible to assess the cell-mediated or humoral (antibody) immune response to these antigens by various methods:

1) Lymphocyte stimulation assays can be performed using varying concentrations of the specific antigens as mitogens in the lymphocyte stimulation microculture system described above. The only difference is that the incubation period is extended to five days from the three used for the polyclonal mitogens.

2) Antibody response to soluble antigens such as BSA or KLH can be quantitated using a micro-ELISA solid phase heterogeneous immunoassay. The assay can be designed to quantitate class specific immunoglobulins (i.e. IgG or IgM).

Results of the humoral (antibody) response to BSA are obtained using a solid phase heterogeneous sandwich ELISA micromethod in 96 well Immulon microtiter plates. The cut-off for the titer was the highest dilution of mouse serum yielding an O.D. $\leq 0.1$ when read on the MR—600 Dynatech micro-ELISA reader.

C. Measurement of Cytotoxic Lymphocyte Activity — Method

A mixed lymphocyte tumour culture (MLTC) immunoassay, used to determine the cytotoxic lymphocytes (CTL) activity, employed lymphocytes from either immunized (sensitized) mice or DBA/2 tumour dormant state animals in various stages of the tumour dormant state (TDS). The assay employed L5178Y target cells labeled with $^{51}$Chromium and effector cells (sensitized lymphocytes) from the above-mentioned sources. Effector-target cell ratios varied with different experimental conditions and may or may not include irradiated stimulator L5178Y cells. Results were expressed as % lysis of target cells based on ratio of cpm of test group over cpm of total release (maximum) both of which are corrected for spontaneous release of $^{51}Cr$. The assay can be performed as an 8 or 18 hour release assay.

In order to induce the tumour dormant state (TDS), a large group of DBA/2 mice was injected subcutaneously with $1 \times 10^6$ viable L5178Y leukaemia cells subcutaneously on the midventral side of the abdomen. Ten days later the resultant tumour nodule, about 1 cm in size, was surgically excised. If the excision was successful, no subcutaneous tumours developed at the site of implantation. Seven days after excision, the mice were challenged with 50,000 viable L5178Y leukaemia cells, a dose that routinely produces death due to ascitic tumours in 100% of normal DBA/2 mice within 14 days. Immune mice resisted rapid outgrowth of the challenge L5178Y dose and remained clinically normal for many weeks thereafter. These mice were considered to be in the tumour dormant state.

D. Anti-cancer Studies — Method

Mice of $BDF_1$ strain were given an i.p. inoculation of $10^6$ L—1210 leukaemia cells on day 0. Twenty-four hours later, groups of 7 mice each were started on a nine day treatment regimen of various doses of the compounds of this invention. The experiment was continued according to the guidelines provided under the National Cancer Institute protocol for screening new anti-cancer agents; T/C ratios (median or mean survival time of treated groups over median or mean survival time of controls) were calculated. A T/C value $\geq 1.25$ (or 125%) is considered to indicate significant anticancer activity.

Preparation of the compounds of the invention is illustrated by the following non-limiting Examples 1 to 6, whilst the remaining Examples illustrate the activities of these compounds.

Example 1

93 g of 2-methyl-2,5-dimethoxy-2,5-dihydrofuran [prepared as described by N. Clauson-Kass and F. Lindborg (1947) *Acta Chem. Scand. 1*: 619] were added under vigorous mechanical stirring to a solution of 75 g of L-ascorbic acid in 750 ml of water at room temperature. The furan was used in 50% excess. The solution became homogenous within 15—20 minutes. The reaction was monitored by a high pressure liquid chromograph equipped with a UV detector. The original peak of Rf = 1.6 of L-ascorbic acid at 254 nm in 20% aqueous methanol disappeared while a new peak appeared at Rf = 5.9 to 6.0, under 64 atmospheres. It was simultaneously observed that the solution did not consume any more iodine, which is indicative of the total disappearance of L-ascorbic acid.

After standing overnight at 20°C, the aqueous solution was freeze-dried to give a pale yellow foam. The latter showed well defined IR, $^{13}C$ NMR and $^1H$ NMR spectra.

Next, 64.0 g (0.25 M) of the crude product (melting point 55°C) and 25.0 g (0.25 M) of succinic anhydride

were placed into a 1 litre round-bottomed flask equipped with a reflux condenser and a nitrogen inlet tube. After adding 200 ml of HPLC-grade ethyl acetate, the reaction mixture was refluxed for 4 hours. The homogeneous solution was cooled to room temperature and then placed in an ice-water bath. A white precipitate formed and this was filtered by suction and washed with a few ml of cold ethyl acetate to give 29.9 g of the crude product (yield 46.6%). The filtrate was concentrated to half volume and cooled in an ice-water bath. The second crop of precipitate was again filtered to give 13.5 g of solid which contained some unreacted succinic anhydride.

The product was recrystallized from a solvent mixture (ethyl acetate/chloroform: 20/80 by volume) to give a total yield of 22.5 g (yield 35.2%) of pure product (long white needles — melting point 134—134.5°C).

Elemental Analysis:

| | | | |
|---|---|---|---|
| Calculated for $C_{26}H_{28}O_{17}$: | C, 50.99%; | H, 4.61%; | O, 44.41%. |
| Found: | C, 50.67%; | H, 4.52%; | O, 44.58%. |

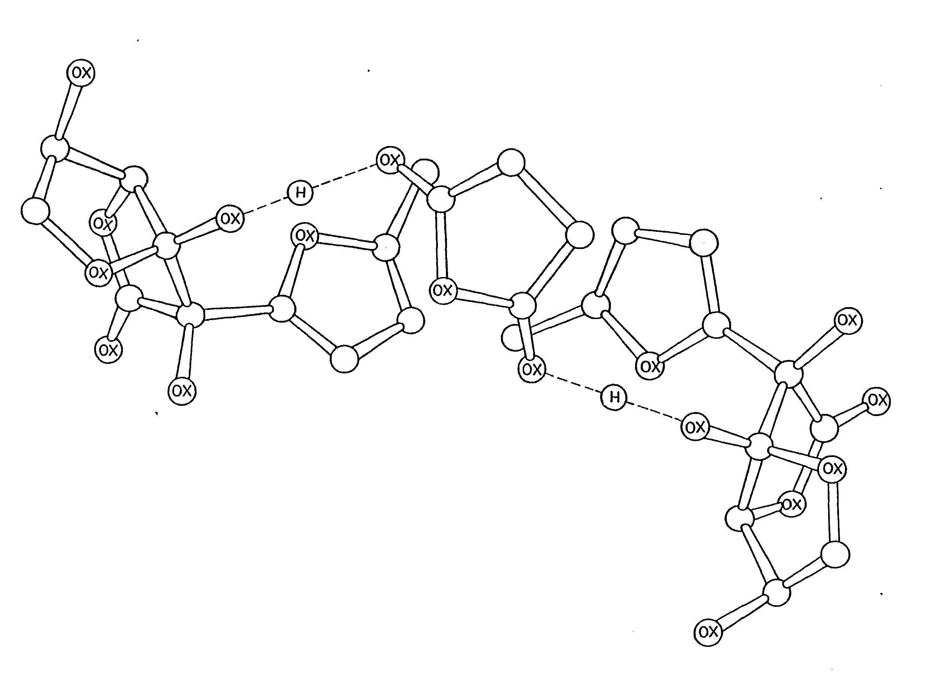

X-ray crystallography confirmed the structure of the compound to be as shown in the accompanying drawing and showed the presence of a 2:1 2-furylbutyrolactone:succinic anhydride, molecular complex in a unit cell, specifically:

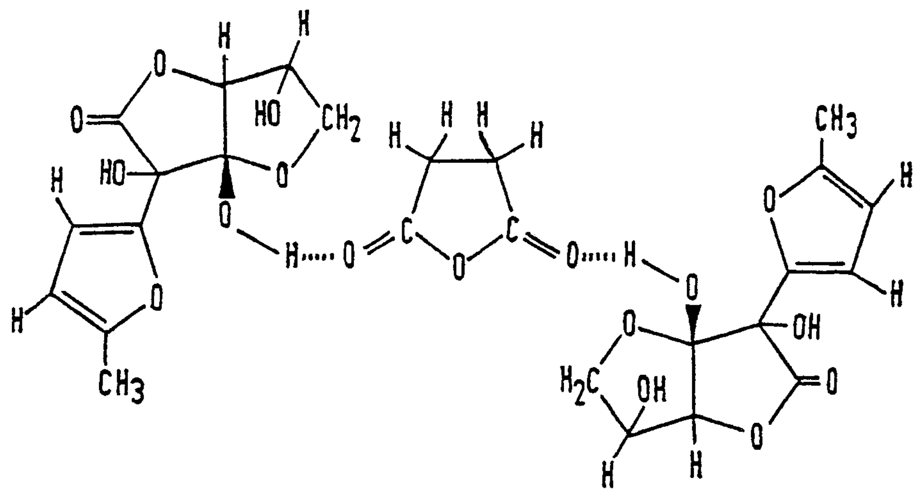

Example 2

186 g of 2-methyl-2,5-2,5-dimethoxy-dihydrofuran were added under vigorous mechanical stirring to a solution of 150 g of L-ascorbic acid in 1.5 litres of water at room temperature. The furan was used in 50% excess. The solution became homogenous within 15—20 minutes. The reaction was monitored by a high pressure liquid chromograph equipped with a UV detector. The original peak of Rf = 1.6 of L-ascorbic acid at 254 nm in 20% aqueous methanol disappeared while a new peak appeared at Rf = 5.9 to 6.0, under 64 atmospheres. It was simultaneously observed that the solution did not consume any more iodine, which is indicative of the total disappearance of L-ascorbic acid.

After standing overnight at 20°C, the aqueous solution was freeze-dried to give a pale yellow foam. The latter showed well defined IR, $^{13}C$ NMR and $^{1}H$ NMR spectra.

Next, 119.65 g (0.467 mole — assuming 100% purity) of the crude product (melting point 55°C) were dissolved in 216 ml of HPLC grade ethyl acetate. 23.3 g succinimide (0.235 mole) were added and the mixture was stirred under positive nitrogen pressure. After a few minutes of stirring, a white solid precipitated. The reaction mixture was then heated for 30 minutes in an oil bath until the solid redissolved. Heating was stopped and the solution was allowed to cool to room temperature while stirring, after which it was cooled in an ice water bath fór 2 hours. A precipitate formed which was filtered by suction, washed with 150 ml of cold chloroform and dried under vacuum to give 67.4 g of crude product (56.3% yield).

The product was recrystallized from a solvent mixture (ethyl acetate/chloroform: 60/40 by volume) to give a total yield of 52.2 g (yield 43.6%) of pure product (long white needles — melting point 132—133°C).

Elemental Analysis:

| | | | | |
|---|---|---|---|---|
| Calculated for $C_{26}H_{29}NO_{16}$: | C, 51.07%; | H, 4.78%; | O, 41.86%; | N, 2.29%. |
| Found: | C, 51.17%; | H, 4.93%; | O, 41.81%; | N, 2.21%. |

X-ray crystallography confirmed the structure of the compound to be as follows and showed the presence of a 2:1 2-furylbutyrolactone:succinimide molecular complex in a unit cell:

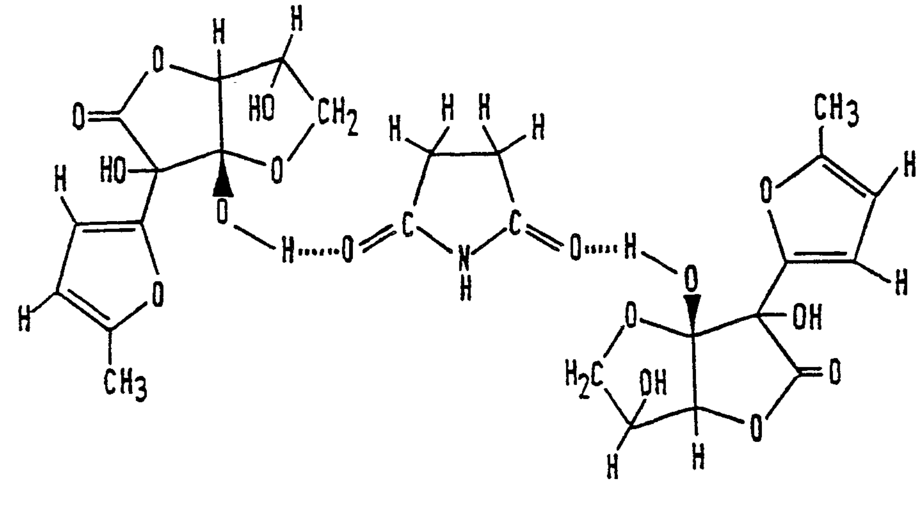

Example 3

93 g of 2-methyl-2,5-dimethoxy-2,5-dihydrofuran were added under vigorous mechanical stirring to a solution of 75 g of L-ascorbic acid in 750 ml of water at room temperature. The furan was used in 50% excess. The solution became homogenous within 15—20 minutes. The reaction was monitored by a high pressure liquid chromograph equipped with a UV detector. The original peak of Rf = 1.6 of L-ascorbic acid at 254 nm in 20% aqueous methanol disappeared while a new peak appeared at Rf = 5.9 to 6.0, under 64 atmospheres. It was simultaneously observed that the solution did not consume any more iodine, which is indicative of the total disappearance of L-ascorbic acid.

After standing overnight at 20°C, the aqueous solution was freeze-dried to give a pale yellow foam. The latter showed well defined IR, $^{13}C$ NMR and $^{1}H$ NMR spectra.

Next, 10.24 g (0.04 M) of the crude product (melting point 55°C) and 2.5 g (0.022 M) of *N*-methylsuccinimide were placed into a 1 litre round-bottomed flask equipped with a reflux condenser and a nitrogen inlet tube. After adding 20 ml of HPLC-grade ethyl acetate, the reaction mixture was refluxed for 4 hours. The homogeneous solution was cooled to room temperature and then placed in an ice-water bath. A white precipitate formed which was filtered by suction and washed with a few ml of cold ethyl acetate to give 5.20 g of the crude product (yield 50.8%). The filtrate was concentrated to half volume and cooled in an ice-water bath.

The product was recrystallized from a solvent mixture (ethyl acetate/chloroform: 1/1 by volume) to give a total yield of 3.21 g (yield 31.3%) of pure product (long white needles — melting point 105—106.5°C).

Elemental Analysis:

| | | | | |
|---|---|---|---|---|
| Calculated for $C_{27}H_{31}NO_{16}$: | C, 51.84%; | H, 5.00%; | N, 2.24%; | O, 40.92%. |
| Found: | C, 51.98%; | H, 5.12%; | N, 2.08%; | O, 40.63%. |

X-ray crystallography confirmed the structure of the compound to be as follows and showed the presence of a 2:1, 2-furylbutyrolactone:*N*-methylsuccinimide molecular complex in a unit cell:

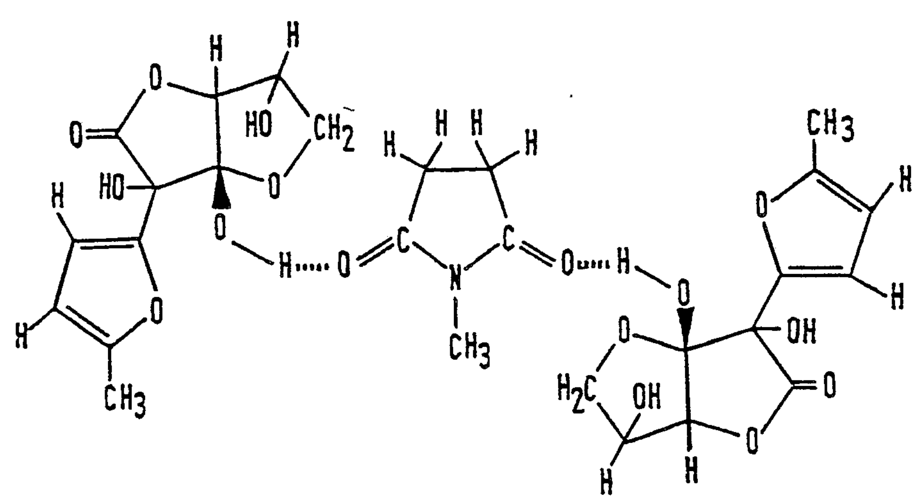

Example 4

The compound of Example 1 dissolved completely in 2.38 mM bicarbonate buffered saline (0.85%) to form

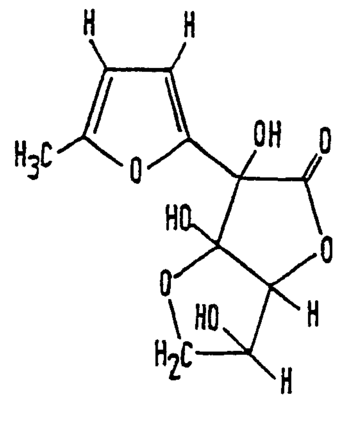

and succinic anhydride.

Example 5

The compound of Example 2 dissolved completely in 2.38 mM bicarbonate buffered saline (0.89%) to form

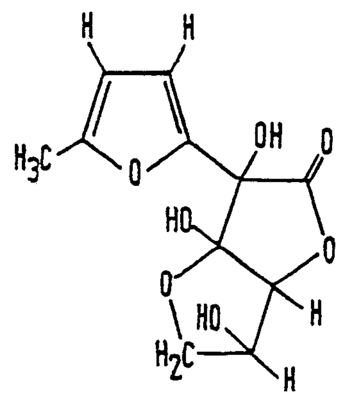

and succinimide.

Example 6

The compound of Example 3 dissolved completely in 2.38 mM bicarbonate buffered saline (0.85%) to form

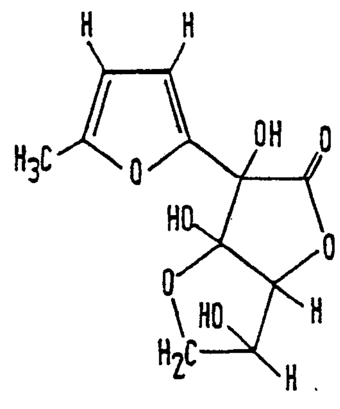

and *N*-methylsuccinimide.

Example 7

Single dose $LD_{50}$ in C57BL/6 mice for Cpd. 1 (i.e. the compound prepared as in Example 1) was determined following the method of Spearman and Karber (Basic Exercises in Immunochemistry, 1979). Briefly, 0.5 ml of each dose was given to each of ten mice. The survivors were counted after 3 days and the $LD_{50}$ was calculated using the following formula:

$$\text{Log } LD_{50} = \log(\text{highest dose tested}) + (\log D)(1/2 - \Sigma R/N)$$

where D = fold differences between doses, R = total number dead, and N = total number of animals tested.

The results are shown in Table 1.

TABLE 1

Results of Cpd.1 Single I. P. Dose $LD_{50}$ Determination Using C57BL/6 Strain of Mice

| Dose of Cpd.1 | Number Dead on Day 3 |
|---|---|
| 272 mg/17g mouse (16,000 mg/kg) | 10 |
| 136 mg/17g mouse (8,000 mg/kg) | 10 |
| 68 mg/17g mouse (4,000 mg/kg) | 7 |
| 34 mg/17g mouse (2,000 mg/kg) | 0 |
| 17 mg/17g mouse (1,000 mg/kg) | 0 |

Cpd.1 $LD_{50}$ = 59.2 mg/17g mouse = 3,480 mg/kg.

Purified Cpd. 1 was tested in C57BL/6 mice by single i.p. injection and gave an $LD_{50}$ in excess of 3 g/kg.

Example 8

Single oral dose $LD_{50}$ in CD—1 mice and CD rats, obtained from Charles River U.K. Ltd., for Cpd. 1 was determined. Briefly, the compound, 5 g/kg, was dissolved in 0.0238 M bicarbonate buffer containing 0.85% sodium chloride, pH 6.8, and administered orally using a constant dose volume of 10 ml/kg. Cpd. 1, dissolved in buffer, or just buffer was administered orally and the mice and rats were observed for 14 days after dosing. After 14 days, the animals were sacrificed and necropsy was performed to check for gross evidence of toxicity.

No deaths or signs of toxicity were observed during this study, indicating a single oral dose $LD_{50}$ for Cpd. 1 in excess of 5 g/kg.

Example 9

Two strains of mice, C 57BL/6 and $BDF_1$ were treated for seven days with i.p. administration of either 25, 50, 100, 200, or 400 mg/kg of Cpd. 1 or just buffer. This was followed in the eighth day by a lymphocyte stimulation assay. The results are shown in Tables 2—A and 2—B.

TABLE 2-A

Effect of Cpd. 1 *In Vivo*, as Measured by the Lymphocyte Stimulation Assay, on C57BL/6 Strain of Mice

| | Lymphocyte Stimulation Index | | |
|---|---|---|---|
| Cpd.1 Conc. | PHA | Con-A | Control |
| 400 mg/kg | 3.2 | 20.7 | 1.0 |
| 200 mg/kg | 21.3 | 34.0 | 1.0 |
| 100 mg/kg | 17.7 | 40.0 | 1.3 |
| 50 mg/kg | N/R | 26.0 | 1.0 |
| 0 mg/kg | 17.3 | 16.7 | --- |

N/R = No results reported

TABLE 2-B

Effect of Cpd. 1 *In Vivo*, as Measured by the Lymphocyte Stimulation Assay, on $BDF_1$ Strain of Mice

| | Lymphocyte Stimulation Index | | |
|---|---|---|---|
| Cpd.1 Conc. | PHA | Con-A | Control* |
| 400 mg/kg | 1.1 | 0.9 | 0.9 |
| 200 mg/kg | 7.8 | 2.9 | 0.8 |
| 100 mg/kg | 48.7 | 17.9 | 0.7 |
| 50 mg/kg | 52.1 | 21.6 | 0.9 |
| 25 mg/kg | 13.8 | 4.3 | 0.8 |
| 0 mg/kg | 5.5 | 1.6 | --- |

* Buffer, pH 8.05

The high dose of 400 mg/kg showed strong suppression of the PHA response in the C57BL/6 mice as well as $BDF_1$ mice, a slight suppression of the Con-A response in the $BDF_1$ mice, and a suppression of the Con-A response in C57BL/6 mice when compared to the 200 mg/kg dose. However, stimulation of T-lymphocyte proliferation was evident for PHA at doses of 50 and 100 mg/kg in $BDF_1$ mice and at a dose of 200 mg/kg in C57BL/6 mice. The Con-A response in the $BDF_1$ strain was significantly increases at doses betwen 50 and 100 mg/kg of Cpd. 1 and in the C57BL/6 strain, it was increased at doses of 50, 100 and 200 mg/kg.

Example 10

A comparison was made of the lymphocyte response of C57BL/6 mice treated for 4 days with i.p. administration of either 50, 100, 200, 400 or 800 mg/kg of Cpd. 1, 50, 100, 200, 400 or 800 mg/kg of ascorbic acid (AA), or just buffer. This was followed on the fifth day by a lymphocyte stimulation assay. The results are shown in Table 3—A and 3—B.

TABLE 3-A

Effect of AA *In Vivo*, as Measured by the Lymphocyte Stimulation Assay, in C57BL/6 Strain of Mice

| | Lymphocyte Stimulation Index | | |
|---|---|---|---|
| AA Conc. | PHA | Con-A | Control |
| 800 mg/kg | 12.1 | 23.9 | 0.50 |
| 400 mg/kg | 14.3 | 17.8 | 1.3 |
| 200 mg/kg | 10.0 | 21.1 | 1.3 |
| 100 mg/kg | 17.1 | 19.8 | 1.8 |
| 50 mg/kg | 17.1 | 25.5 | 0.56 |
| 0 mg/kg | 14.2 | 17.1 | --- |

TABLE 3-B

Effect of Cpd. 1 *In Vivo*, as Measured by the Lymphocyte Stimulation Assay, in C57BL/6 Strain of Mice

| | Lymphocyte Stimulation Index | | |
|---|---|---|---|
| Cpd.1 Conc. | PHA | Con-A | Control |
| 800 mg/kg | 0.408 | 0.612 | 0.31 |
| 400 mg/kg | 4.8 | 40.0 | 1.0 |
| 200 mg/kg | 42.8 | 67.4 | 1.6 |
| 100 mg/kg | 34.8 | 80.0 | 1.6 |
| 50 mg/kg | N/R | 52.8 | 1.2 |
| 0 mg/kg | 33.0 | 32.6 | --- |

No statistically significant measureable effect of AA was demonstrable with PHA but a single stimulation of proliferation did occur at 50 mg/kg in the Con-A stimulated group.

In spleen cells treated with PHA, doses of 100 and 200 mg/kg of Cpd. 1 tended to stimulate lymphocyte DNA synthesis. Doses ranging from 400 to 800 suppressed DNA synthesis of spleen lymphocytes. In spleen cells treated with Con-A doses ranging from 50 to 200 mg/kg had a significant stimulatory effect and doses ranging from 400 to 800 mg/kg had a significant anti-proliferative effect, particularly the 800 mg/kg dose. These data suggest an immunostimulatory effect of Cpd. 1 at low to median concentrations and an immunosuppressive effect of Cpd. 1 at higher concentrations. Also, the possibility that ascorbic acid by itself could exert a similar effect to Cpd. 1 was unsupported by these experiments.

Example 11

Two strains of mice, C57BL/6 and BALB/c, were treated for 4 days with i.p. administration of either 50, 100, 200, or 400 mg/kg of Cpd. 1, or just buffer. This was followed on the fifth day by a lymphocyte stimulation assay. The results are shown in Tables 4—A and 4—B.

TABLE 4-A

Effect of Cpd. 1 *In Vivo*, as Measured by the Lymphocyte Stimulation Assay, on C57BL/6 Strain of Mice

| | Lymphocyte Stimulation Index | | |
|---|---|---|---|
| Cpd.1 Conc. | PHA | Con-A | Control |
| 400 mg/kg | 14.8 | 30.9 | 0.61 |
| 200 mg/kg | 25.2 | 41.1 | 0.93 |
| 100 mg/kg | 17.0 | 22.3 | 1.3 |
| 50 mg/kg | 13.6 | 20.6 | 0.87 |
| 0 mg/kg | 7.85 | 11.4 | --- |

TABLE 4-B

Effect of Cpd. 1 *In Vivo*, as Measured by the Lymphocyte Stimulation Assay, on BALB/C Strain of Mice

| | Lymphocyte Stimulation Index | | |
|---|---|---|---|
| Cpd.1 Conc. | PHA | Con-A | Control |
| 400 mg/kg | 6.71 | 18.7 | 0.90 |
| 200 mg/kg | 7.65 | 20.5 | 0.76 |
| 100 mg/kg | 5.24 | 21.8 | 1.0 |
| 50 mg/kg | 6.44 | 20.5 | 1.4 |
| 0 mg/kg | 1.76 | 9.87 | --- |

In spleens treated with PHA and Con-A, doses of 50, 100 and 200 mg/kg tended to stimulate lymphocyte DNA synthesis in both strains of mice. Doses of 400 mg/kg suppressed DNA synthesis of spleen cell lymphocytes when compared to the activity at a dose of 200 mg/kg.

Example 12

An *in vitro* lymphocyte stimulation assay was performed directly on normal spleen lymphocytes removed from five normal C57BL/6 mice, using concentrations of Cpd. 1 of 1, 10, $10^2$, $10^3$ or $10^4$ ng per test well ($5 \times 10^5$ cells/well) or just buffer. Following three days of incubation the assay was completed. The results are shown in Table 5.

TABLE 5

Effect of Cpd. 1 *In Vivo*, as Measured by the Lymphocyte Stimulation Assay, in C57BL/6 Strain of Mice

| | Lymphocyte Stimulation Index | | |
|---|---|---|---|
| Cpd.1 Conc. | PHA | Con-A | Control |
| $10^4$ ng/well | 0.920 | 2.15 | 1.9 |
| $10^3$ ng/well | 53.8 | 178 | 2.6 |
| $10^2$ ng/well | 83.1 | 198 | 3.2 |
| 10 ng/well | 87.5 | 198 | 3.2 |
| 1 ng/well | 80.4 | 198 | 3.8 |
| 0 ng/well | 21.9 | 126 | --- |

The results indicate significant stimulation of mitogenesis across a range of doses from 1.0—$10^3$ ng/well and significant suppression at a dose of $10^4$ ng/well for both PHA and Con-A mitogens. The range of concentration effectiveness *in vitro* may be the result of exclusion of the influence of the host's metabolic system on drug metabolism.

Example 13

Lymphocytes isolated from 5 human volunteers were treated *in vitro* with PHA and Con-A and either $10^2$, $10^3$, $10^4$, $5 \times 10^4$ or $10^5$ ng/well of Cpd. 1, $10^2$, $10^3$, $10^4$, $5 \times 10^4$, or $10^5$ ng/well of AA, or buffer. The results are shown in Tables 6—A and 6—B.

TABLE 6-A

Effect of AA on the *In Vitro* Lymphocyte Stimulation Assay in Humans

| | Lymphocyte Stimulation Index | |
|---|---|---|
| AA Concentration | PHA | Con-A |
| $10^5$ ng/well | 34.23 | 25.18 |
| $5\times10^4$ ng/well | 35.93 | 23.31 |
| $10^4$ ng/well | 35.10 | 27.35 |
| $10^3$ ng/well | 34.69 | 24.76 |
| $10^2$ ng/well | 38.29 | 24.37 |
| 0 ng/well | 31.25 | 25.86 |

TABLE 6-B

Effect of Cpd. 1 on the *In Vitro* Lymphocyte Stimulation Assay in Humans

| | Lymphocyte Stimulation Index | |
|---|---|---|
| Cpd.1 Concentration | PHA | Con-A |
| $10^5$ ng/well | 0.544 | 0.44 |
| $5\times10^4$ ng/well | 0.40 | 1.33 |
| $10^4$ ng/well | 34.37 | 23.82 |
| $10^3$ ng/well | 48.60 | 32.63 |
| $10^2$ ng/well | 56.67 | 38.06 |
| 0 ng/well | 39.82 | 26.62 |

There is no significant proliferative or anti-proliferative effect demonstrated for AA alone, whereas for Cpd. 1, a high dose anti-proliferative effect, at doses of $10^5$ and $5 \times 10^4$ ng/ml, was followed by a markedly increased lymphocyte response at very low doses of $10^3$ and $10^2$ ng/ml. The same profiles of results were exhibited for both PHA and Con-A mitogens.

Example 14

C57BL/6 mice were treated with four daily i.p. doses of either 50, 100, 200, or 400 mg/kg of Cpd. 1 or just buffer. On the fifth day a lymphocyte stimulation assay was performed. The results are shown in Table 7.

TABLE 7

Effect of Cpd. 1 *In Vivo*, as Measured by the Lymphocyte Stimulation Assay, in C57BL/6 Strain of Mice

| | Lymphocyte Stimulation Index | | |
|---|---|---|---|
| Cpd.1 Conc. | PHA | Con-A | Control |
| 400 mg/kg | 18.7 | 26.4 | 0.31 |
| 200 mg/kg | 23.2 | 26.3 | 0.22 |
| 100 mg/kg | 23.8 | 25.4 | 0.40 |
| 50 mg/kg | 17.0 | 23.0 | 1.1 |
| 0 mg/kg | 14.9 | 19.9 | --- |

Stimulation of the response to polyclonal mitogens at doses of Cpd. 1 from 50—400 mg/kg with a maximum response achieved between doses of 100—200 mg/kg for both PHA and Con-A test mitogens was observed. It can also be seen that there was a decrease in the response to PHA at the 400 mg/kg dose when compared to the response at the 200 mg/kg dose. This appears to follow the trend of immunosuppression at high doses.

Example 15

The *in vivo* immunomodulatory effect of Cpd. 1 on C57BL/6 mice was studied.

Mice of the C57BL/6 strain were tested with i.p. administration of either 100, 200, 400 or 600 mg/kg of Cpd. 1 or just buffer. This was followed by removal of spleens and treatment of lymphocytes therein with mitogens PHA and Con-A. The results are shown in Table 8.

TABLE 8

Effect of Cpd. 1 *In Vivo*, as Measured by the Lymphocyte Stimulation Assay, on C57BL/6 Strain of Mice

| | Lymphocyte Stimulation Index | | |
|---|---|---|---|
| Cpd.1 Conc. | PHA | Con-A | Control |
| 600 mg/kg | 17.6 | 26.5 | 1.7 |
| 400 mg/kg | 44.9 | 42.4 | 0.49 |
| 200 mg/kg | 48.2 | 36.6 | 0.62 |
| 100 mg/kg | 51.1 | 43.9 | 0.80 |
| 0 mg/kg | 13.1 | 26.7 | --- |

Doses ranging from 100 to 400 mg/kg of Cpd. 1 caused significant stimulation of both the Con-A and PHA treated lymphocytes. The high dose, 600 mg/kg, caused less stimulation than the 100 to 400 mg/kg doses and gave levels comparable to controls. There was no evidence of toxicity in the 600 mg/kg treated animals given the five day administration of Cpd. 1.

Example 16

An *in vitro* lymphocyte stimulation assay was performed using spleen lymphocytes from normal C57BL/6 mice. The lymphocytes were treated with either $10^3$, $5 \times 10^3$, $10^4$, $5 \times 10^4$, or $10^5$ ng/well of Cpd. 1 or buffer. The results are shown in Table 9.

TABLE 9

Effect of Cpd. 1 *In Vivo*, as Measured by the Lymphocyte Stimulation Assay, on C57BL/6 Strain of Mice

| | Lymphocyte Stimulation Index | | |
|---|---|---|---|
| Cpd.1 Conc. | PHA | Con-A | Control |
| $10^5$ ng/well | 0.292 | 0.308 | 0.20 |
| $5 \times 10^4$ ng/well | 0.261 | 3.78 | 0.20 |
| $10^4$ ng/well | 126 | 194 | 0.66 |
| $5 \times 10^3$ ng/well | 157 | 162 | 1.8 |
| $10^3$ ng/well | 80.4 | 123 | 1.2 |
| 0 ng/well | 59.5 | 122 | --- |

Doses of $5 \times 10^3$ and $10^4$ ng/well caused significant increases in the response to PHA and Con-A while doses of $5 \times 10^4$ and $10^5$ ng/well caused significant decrease in the lymphocyte response to the mitogens.

Example 17

The compound of Example 2, Cpd. 2, was tested *in vivo* by using C57BL/6 mice and treating them with Cpd. 2 or just buffer for four consecutive days by the i.p. route. On the fifth day a lymphocyte stimulation assay was performed. The results are shown in Table 10.

TABLE 10

Effect of Cpd. 2 *In Vivo*, as Measured by the Lymphocyte Stimulation Assay, on C57BL/6 Strain of Mice

| | Lymphocyte Stimulation Index | | |
|---|---|---|---|
| Cpd.2 Conc. | PHA | Con-A | Control |
| 600 mg/kg | 15.8 | 94.0 | 1.5 |
| 400 mg/kg | 12.9 | 109 | 1.3 |
| 200 mg/kg | 11.3 | 99.3 | 0.69 |
| 100 mg/kg | 15.4 | 105 | 1.5 |
| 50 mg/kg | 15.5 | 96.1 | 0.88 |
| 0 mg/kg | 11.0 | 75.1 | --- |

Example 18

Cpd. 2 was tested using normal spleen lymphocytes from C57BL/6 mice. A lymphocyte stimulation assay was conducted using either $10^3$, $5 \times 10^3$, $10^4$O, $5 \times 10^4$, or $10^5$ ng/well Cpd. 2 or just buffer. The results are shown in Table 11.

TABLE 11

Effect of Cpd. 2 *In Vivo*, as Measured by the Lymphocyte Stimulation Assay, on C57BL/6 Strain of Mice

| | Lymphocyte Stimulation Index | | |
|---|---|---|---|
| Cpd.2 Conc. | PHA | Con-A | Control |
| $10^5$ ng/well | 3.83 | 144 | 0.49 |
| $5\times10^4$ ng/well | 45.8 | 218 | 0.80 |
| $10^4$ ng/well | 38.8 | 164 | 1.1 |
| $5\times10^3$ ng/well | 29.8 | 148 | 1.2 |
| $10^3$ ng/well | 25.4 | 147 | 0.92 |
| 0 ng/well | 23.5 | 175 | --- |

In spleen cells treated with PHA, doses of $5 \times 10^4$ and $10^5$ ng/well of Cpd. 2 tended to stimulate lymphocyte DNA synthesis. The 105 ng/well dose suppressed DNA synthesis of spleen lymphocytes. In spleen cells treated with Con-A, a $2 \times 10^5$ ng/well dose had a stimulatory effect and a $4 \times 10^5$ ng/well dose had an anti-proliferative effect.

Example 19

Spleen cells from C57BL/6 mice were treated, *in vitro*, with the crystallizing reagents, succinic acid and succinimide. Since these compounds were spontaneously released from the molecular complex in solution at a molar ratio of 2 moles of Cpds. 1, 2, or 3 per mole of the crystalline reagent, these reagents were treated in the lymphocyte stimulation assay at proper molar concentrations. The results are shown in Tables 12—A and 12—B.

TABLE 12-A

Effect of Succinic Acid *In Vivo*, as Measured by the Lymphocyte Stimulation Assay, in C57BL/6 Strain of Mice

| Lymphocyte Stimulation Index | | | |
|---|---|---|---|
| Succinic Acid Conc. | PHA | | Con-A |
| Control | | | |
| $1.66 \times 10^4$ ng/well | 11.5 | 38.5 | 1.1 |
| $8.3 \times 10^3$ ng/well | 12.4 | 35.6 | 0.91 |
| $4.15 \times 10^3$ ng/well | 14.0 | 42.3 | 0.91 |
| $2.08 \times 10^3$ ng/well | 15.3 | 51.1 | 1.0 |
| $1.04 \times 10^3$ ng/well | 15.7 | 64.6 | 0.94 |
| 0 ng/well | 15.9 | 83.5 | --- |

TABLE 12-B

Effect of Succinimide *In Vivo*, as Measured by the Lymphocyte Stimulation Assay, in C57BL/6 Strain of Mice

| Lymphocyte Stimulation Index | | | |
|---|---|---|---|
| Succinimide Conc. | PHA | | Con-A |
| Control | | | |
| $1.392 \times 10^4$ ng/well | 17.1 | 92.9 | 1.4 |
| $6.96 \times 10^3$ ng/well | 17.2 | 71.9 | 1.1 |
| $3.48 \times 10^3$ ng/well | 18.1 | 85.1 | 0.98 |
| $1.74 \times 10^3$ ng/well | 17.5 | 83.4 | 1.1 |
| $8.7 \times 10^2$ ng/well | 19.5 | 76.0 | 0.91 |
| 0 ng/well | 19.1 | 100 | --- |

The lymphocytes treated with succinic acid showed a decrease in PHA and Con-A mitogenesis with increased succinic acid dosage. The succinimide demonstrated no significant suppressive or enhancing effect on the response of the lymphocytes to either mitogen.

Example 20

The compound of Example 3, Cpd. 3, was used to treat C57BL/6 spleen lymphocytes *in vitro*. The lymphocytes were treated with either $10^3$, $5 \times 10^3$, $10^4$, $5 \times 10^4$ or $10^5$ ng/well of Cpd. 3 or just buffer and then a lymphocyte stimulation assay was performed. The results are shown in Table 13.

TABLE 13

Effect of Cpd. 3 *In Vivo*, as Measured by the Lymphocyte Stimulation Assay, on C57BL/6 Strain of Mice

| | Lymphocyte Stimulation Index | | |
|---|---|---|---|
| Cpd.3 Conc. | PHA | Con-A | Control |
| $10^5$ ng/well | 0.263 | 2.88 | 0.25 |
| $5x10^4$ ng/well | 4.08 | 97.2 | 0.27 |
| $10^4$ ng/well | 24.0 | 89.6 | 0.47 |
| $5x10^3$ ng/well | 18.5 | 80.9 | 0.64 |
| $10^3$ ng/well | 12.5 | 73.2 | 0.60 |
| 0 ng/well | 12.2 | 94.6 | --- |

The $10^4$ ng/well dose of Cpd. 3 significantly stimulated the response to PHA while the $5 \times 10^4$ and $10^5$ ng/well doses suppressed the response. The $5 \times 10^4$ ng/well dose slightly stimulated the response to Con-A and the $10^5$ ng/well dose suppressed the response.

Example 21

Three month old mice of the SJL/J strain, which are T-cell deficient, are treated with i.p. administration of 100, 200, or 400 mg/kg of Cpd. 1 or just buffer for four days. This was followed by a lymphocyte stimulation assay. The results are shown in Table 14.

TABLE 14

Effect of Cpd. 1 *In Vivo*, as Measured by the Lymphocyte Stimulation Assay, in SJL/J Strain of Mice

| | Lymphocyte Stimulation Index | | |
|---|---|---|---|
| Cpd.1 Conc. | PHA | Con-A | Control |
| 400 mg/kg | 2.31 | 3.30 | 0.693 |
| 200 mg/kg | 1.79 | 4.57 | 0.732 |
| 100 mg/kg | 1.85 | 5.01 | 0.573 |
| 0 mg/kg | 1.11 | 2.4 | --- |

Example 22

SJL/J mice apparently shift to a loss of immune regulation function as they age and the latter is evidenced by a hyper-responsiveness to alloantigens and production of circulating antibodies to nuclear material as well as synthetic double stranded RNA and poly I/C. The continuous erosion of immune competence in the SJL/J mice has been observed to extend from birth to death indicating defects in regulatory T-cell subpopulations (possibly suppressor and/or amplifier cells). Because the dynamics of the T-lymphocyte population changes with age, it must be assumed that age plays a role in affecting the results obtained when treating SJL/J mice with Cpd. 1.

An *in vivo* lymphocyte stimulation assay was performed on SJL/J mice of different ages, either 2, 5, or 10 months. The mice received i.p. injections of either 100 mg/kg of Cpd. 1 or just buffer. The results are shown in Table 15.

TABLE 15

Effect of Cpd. 1 *In Vivo*, as Measured by the Lymphocyte Stimulation Assay, in SJL/J Strain of Mice

| Age of Mice (Months) | Cpd.1 Conc. | Lymphocyte Stimulation Index PHA | Con-A | Control |
|---|---|---|---|---|
| 2 | 0 mg/kg | 40.8 | 103 | --- |
| | 100 mg/kg | 32.6 | 167 | 2.3 |
| 5 | 0 mg/kg | 2.09 | 19.5 | --- |
| | 100 mg/kg | 1.16 | 12.9 | 1.9 |
| 10 | 0 mg/kg | 3.97 | 21.9 | --- |
| | 100 mg/kg | 9.18 | 14.5 | 2.1 |

The results obtained from the untreated controls demonstrated the age-dependent variation of the PHA and Con-A lymphocyte proliferation responses.

Also, at 2 months of age, Cpd. 1 administered at a dose of 100 mg/kg caused a decreased PHA versus increased Con-A response. In contrast, at 5 months of age, the SJL/J mice given the dose of Cpd. 1 showed a depressed mitogenesis response to both PHA and Con-A. The ten month old SJL/J mice showed a complete reversal and demonstrated significant stimulation of both the PHA and Con-A mitogenic response upon treatment of mice at the same dose.

Example 23

Mice of the C57BL/6 strain were given i.p. injections of either 50, 100, 200 or 400 mg/kg of Cpd. 1 or buffer for twelve days (days 1—12). An antigen, bovine serum albumin (BSA), was administered intraperitoneally with Freund's Complete Adjuvant (FAC) on days, 1, 7 and 14. Blood samples were taken for assay of the antibody response on days 0, 5, 13 and 19. Also, on days 13 and 10 a lymphocyte proliferation assay was performed on spleen lymphocytes from these same mice using either the BSA antigen as the specific recall antigen (mitogen) at concentrations of 10, $10^2$, $5 \times 10^2$ or $10^3$ ng/well or just buffer. The results are shown in Tables 16—A, 16—B and 16—C.

TABLE 16-A

Enhancement of the Secondary Immune Response to BSA by Cpd. 1, as Measured by Micro-ELISA titer

| Cpd.1.Conc. | Prebleed | Micro-ELISA Titer Day 5 | Day 13 | Day 19 |
|---|---|---|---|---|
| 400 mg/kg | n/r | n/r | 1:80 | 1:320 |
| 200 mg/kg | n/r | n/r | 1:80 | 1:1280 |
| 100 mg/kg | n/r | n/r | 1:40 | 1:1280 |
| 50 mg/kg | n/r | n/r | 1:40 | 1:640 |
| 0 mg/kg | n/r | n/r | 1:20 | 1:320 |

n/r = no response

TABLE 16-B

Effect of Cpd. 1 *In Vivo* on the Secondary Immune Response to BSA, as Measured by Lymphocyte Stimulation Assay, in C57BL/6 Strain of Mice, Day 13

| | Lymphocyte Stimulation Index | | | | |
|---|---|---|---|---|---|
| | BSA (ng/well) | | | | |
| Cpd.1 Conc. | 0 | 10 | $10^2$ | $5\times10^2$ | $10^3$ |
| 400 mg/kg | 10.9 | 13.7 | 12.8 | 12.3 | 13.8 |
| 200 mg/kg | 10.6 | 14.1 | 15.8 | 13.8 | 14.0 |
| 100 mg/kg | 3.22 | 9.8 | 11.9 | 12.3 | 10.2 |
| 50 mg/kg | 16.5 | 19.9 | 23.1 | 22.9 | 18.2 |
| 0 mg/kg | --- | 14.0 | 14.6 | 12.8 | 13.0 |

TABLE 16-C

Effect of Cpd. 1 *In Vivo* on the Secondary Immune Response to BSA, as Measured by Lymphocyte Stimulation Assay, in C57BL/6 Strain of Mice, Day 19

| | Lymphocyte Stimulation Index | | | | |
|---|---|---|---|---|---|
| | BSA (ng/well) | | | | |
| Cpd.1 Conc. | 0 | 10 | $10^2$ | $5\times10^2$ | $10^3$ |
| 400 mg/kg | 5.0 | 3.26 | 2.89 | 1.94 | 1.04 |
| 200 mg/kg | 0.42 | 2.94 | 2.82 | 2.56 | 2.12 |
| 100 mg/kg | 4.1 | 3.79 | 2.68 | 2.03 | 0.26 |
| 50 mg/kg | 0.26 | 2.33 | 2.27 | 2.37 | 2.63 |
| 0 mg/kg | --- | 1.58 | 1.56 | 2.07 | 1.02 |

No antibody response was observed until day 13. On that day, a significant (four-fold) stimulation was observed at Cpd. 1 doses of 200 and 400 mg/kg and only a slight amount of stimulation was observed at doses of 50 and 100 mg/kg of Cpd. 1. A much more dramatic response was observed on day 19. At that time, the antibody responses to BSA were equal at the 0 mg/kg and 400 mg/kg dose of Cpd. 1, and higher than observed on day 13. A slight increase of antibody titer over the control was observed at 50 mg/kg of Cpd. 1 while 100 and 200 mg/kg doses of Cpd. 1 were extremely stimulatory, demonstrating a 16-fold increase over controls.

The second component of Example 23 illustrates the effects of Cpd. 1 on the lymphocyte proliferation of spleen cells to the immunizing antigen (BSA). Only the 50 mg/kg dose shows significant stimulation of DNA synthesis on day 13 compared to controls. This effect had waned by day 19, indicating the importance of sustaining treatment to simulate the cell-mediated (T-cell) component of the immune response. The above mentioned antibody response is likely to be sustained after cessation of therapy since the half life of IgG (the antibody) *in vivo* is approximately 5—7 days.

Example 24

Mice of the C57BL/6 strain were given i.p. injections of either 50, 100, 200 or 400 mg/kg of Cpd. 1 or buffere for ten days (days 1—10). An antigen, keyhole limpet haemocyanin (KLH), was administered intraperitoneally with Freund's Complete Adjuvant (FCA) on days, 1, 7, and 13. Blood samples were taken

for assay of the antibody response on days 0, 5, 12, and 17. Spleens were removed, lymphocytes isolated and a lymphocyte stimulation assay with PHA, Con-A or KLH antigen as the mitogens was performed on day 17 of the experiment. The results are shown in Tables 17—A and 17—B.

TABLE 17-A

Enhancement of the Secondary Immune Response to KLH by Cpd. 1, as Measured by Micro-ELISA Titer

| | Micro-ELISA Titer | | | |
|---|---|---|---|---|
| Cpd.1.Conc. | Prebleed | Day 5 | Day 12 | Day 17 |
| 400 mg/kg | n/r | n/r | 1:5120 | 1:10,240 |
| 200 mg/kg | n/r | n/r | 1:5120 | 1:10,240 |
| 100 mg/kg | n/r | n/r | 1:2560 | 1:5120 |
| 50 mg/kg | n/r | n/r | 1:2560 | 1;5120 |
| 0 mg/kg | n/r | n/r | 1:2560 | 1:1280 |

TABLE 17-B

Effect of Cpd. 1 *In Vivo* on the Secondary Immune Response to KLH, PHA and Con-A as Measured by Lymphocyte Stimulation Assay, in C57BL/6 Strain of Mice, Day 17

| | Lymphocyte Stimulation Index | | | |
|---|---|---|---|---|
| | KLH (ng/well) | | | |
| Cpd.1 Conc. | $10^3$ | $5x10^2$ | 50 | 25 |
| 400 mg/kg | 0.589 | 0.232 | 0.667 | 1.03 |
| 200 mg/kg | 7.45 | 4.26 | 1.02 | 3.91 |
| 100 mg/kg | 0.898 | 0.734 | 0.526 | 0.401 |
| 50 mg/kg | 0.536 | 0.415 | 0.357 | 0.0386 |
| 0 mg/kg | 0.715 | 0.614 | 0.739 | 1.06 |

| Cpd.1 Conc. | PHA | Con-A | Control |
|---|---|---|---|
| 400 mg/kg | 2.32 | 35.5 | 0.840 |
| 200 mg/kg | 12.4 | 51.7 | 1.73 |
| 100 mg/kg | 9.12 | 15.6 | 0.961 |
| 50 mg/kg | 7.65 | 19.0 | 0.976 |
| 0 mg/kg | 11.0 | 34.1 | --- |

No antibody response was observed until day 12, when the 200 and 400 mg/kg treatment groups showed significant stimulation (four-fold increases) over the control treatment groups. A similar pattern of difference between the controls and treated groups held on day 17, seven days after cessation of Cpd. 1 treatment, with the 200 and 400 mg/kg doses showing an eight-fold increase over the untreated antigen-sensitized controls.

The lymphocyte proliferation assay was only performed on day 17, some five days subsequent to completion of the Cpd. 1 treatment protocol. A statistically significant increase in lymphocyte DNA synthesis was seen in the 200 mg/kg treated mice stimulated by Con-A or KLH mitogens. The lymphocyte mitogen response to Con-A (T-cell mitogen) correlated well with a positive response to KLH of the KLH sensitized spleen lymphocytes.

Example 25

The effect of Cpd. 1 on cytotoxic lymphocyte (CTL) activity was measured using five DBA/2 mice in the early stage of the TDS, 10 days after challenge with viable L5178Y tumor cells. Six doses of Cpd. 1, 1, 10, $10^2$, $10^3$, $10^4$, or $10^5$ ng/ml or just buffer were added in 0.1 ml volumes to MLTC microculture wells containing washed spleen lymphocytes from the DBA/2 mice, effector, and L5178Y target cells at varying E/T ratios. The MLTC reaction was performed in the presence or absence of irradiated L5178Y stimulator cells. The results are shown in Tables 18—A and 18—B.

TABLE 18-A

Effect of Cpd. 1 on CTL Activity of TDS Spleen Lymphocytes from DBA/2 Mice, as Measured by MLTC Assay Using Effector Cells Co-cultured with Stimulator Cells

| | % Lysis | | |
|---|---|---|---|
| | E/T Ratio | | |
| Cpd.1 Conc. | 100:1 | 30:1 | 10.1 |
| $10^5$ ng/ml | 87.3 | 54.2 | 21.2 |
| $10^4$ ng/ml | 74.3 | 42.0 | 15.9 |
| $10^3$ ng/ml | 71.5 | 46.0 | 15.6 |
| $10^2$ ng/ml | 79.0 | 51.0 | 20.2 |
| 10 ng/ml | 77.1 | 55.8 | 20.6 |
| 1 ng/ml | 71.1 | 40.2 | 16.7 |
| 0 ng/ml | 55.2 | 23.0 | 8.2 |

TABLE 18-B

Effect of Cpd. 1 on CTL Activity of TDS Spleen Lymphocytes from DBA/2 Strain of Mice, as Measured by MLTC Assay Using Effector Cells Without Stimulator Cells

| Cpd. 1 Conc. | % Lysis E/T Ratio 100:1 | 30:1 | 10.1 |
|---|---|---|---|
| $10^5$ ng/ml | 42.0 | 18.7 | 8.6 |
| $10^4$ ng/ml | 36.3 | 16.8 | 4.6 |
| $10^3$ ng/ml | 40.1 | 20.0 | 4.6 |
| $10^2$ ng/ml | 31.6 | 14.8 | 3.5 |
| 10 ng/ml | 26.0 | 15.7 | 3.5 |
| 1 ng/ml | 23.4 | 12.2 | 3.9 |
| 0 ng/ml | 22.7 | 11.0 | -2.2 |

Cpd. 1 caused a significant increase in CTL activity expressed as % lysis at several concentrations irrespective of the E/T ratio employed or whether or not stimulator cells were present.

This data therefore supports amplification of the specific CTL response of TDS mouse spleen cells sensitized to L5178Y tumor cells in a drug dose dependent manner.

Example 26

Five DBA/2 mice in the early stage of the TDS, 10 days after challenge with viable L5178Y tumor cells were used to measure the effect of Cpd. 1 on CTL activity. Two doses of Cpd. 1, $10^4$, or $10^5$ ng/ml, or just buffer were added in 0.1 ml volumes to the MLTC microculture wells containing washed spleen lymphocytes from the DBA/2 mice, effector and L5178Y target cells at varying E/T ratios. The MLTC reaction was performed in the presence or absence of irradiated L5178Y stimulator cells. The results are shown in Tables 19—A and 19—B.

TABLE 19-A

Effect of Cpd. 1 on CTL Activity of TDS Spleen Lymphocytes from DBA/2 Strain of Mice, as Measured by MLTC Assay Using Effector Cells Co-cultured with Stimulator Cells

| Cpd. 1 Conc. | % Lysis E/T Ratio 50:1 | 25:1 | 12.5:1 | 6.25:1 |
|---|---|---|---|---|
| $10^5$ ng/ml | 71.7 | 79.8 | 75.7 | 56.3 |
| $10^4$ ng/ml | 75.7 | 72.0 | 67.1 | 60.5 |
| 0 ng/ml | 63.9 | 61.9 | 45.0 | 35.5 |

TABLE 19-B

Effect of Cpd. 1 on CTL Activity of TDS Spleen Lymphocytes from DBA/2 Strain of Mice, as Measured by MLTC Assay Using Effector Cells Without Stimulator Cells

| | % Lysis | | | |
|---|---|---|---|---|
| | E/T Ratio | | | |
| Cpd.1 Conc. | 50:1 | 25:1 | 12.5:1 | 6.25:1 |
| $10^5$ ng/ml | 21.8 | 16.6 | 11.1 | 6.1 |
| $10^4$ ng/ml | 12.8 | 8.1 | 6.9 | 0.7 |
| 0 ng/ml | -7.5 | -3.0 | -3.5 | -1.3 |

Cpd 1 caused a significant increase in CTL activity expressed as % lysis, at several concentrations irrespective of the E/T ratio employed or whether or not stimulator cells were present.

Example 27

DBA/2 mice in the early stage of TDS, prior to challenge with viable L5178Y tumour cells, were used to measure the effect of Cpd. 2 on CTL activity. Cpd. 2, $10^2$, $10^3$, $10^4$ or $10^5$ ng/ml, or just buffer was added in 0.1 ml volumes to MLTC microculture wells containing washed spleen lymphocytes from DBA/2 mice, effector, and L5178Y target cells at varying E/T ratios. The MLTC reaction was performed in the presence or absence or irradiated L5178Y stimulator cells. The results are shown in Tables 20—A and 20—B.

TABLE 20-A

Effect of Cpd. 2 on CTL Activity of Pre-Challenge TDS Spleen Lymphocytes from DBA/2 Mice, as Measured by MLTC Assay Using Effector Cells Co-cultured with Stimulator Cells

| | % Lysis | | | |
|---|---|---|---|---|
| | E/T Ratio | | | |
| Cpd.2 Conc. | 100:1 | 50:1 | 25:1 | 12.5:1 |
| $10^5$ ng/ml | 78.5 | 73.5 | 65.0 | 47.0 |
| $10^4$ ng/ml | 73.5 | 72.5 | 72.5 | 58.5 |
| $10^3$ ng/ml | 75.5 | 72.5 | 69.5 | 58.0 |
| $10^2$ ng/ml | 74.5 | 69.5 | 64.0 | 47.5 |
| 0 ng/ml | 68.5 | 67.0 | 57.0 | 37.5 |

TABLE 20-B

Effect of Cpd. 2 on CTL Activity of Pre-Challenge TDS Spleen Lymphocytes from DBA/2 Mice, as Measured by MLTC Assay Using Effector Cells Co-cultured Without Stimulator Cells

| | % Lysis | | | |
|---|---|---|---|---|
| | E/T Ratio | | | |
| Cpd. 2 Conc. | 100:1 | 50:1 | 25:1 | 12.5:1 |
| $10^4$ ng/ml | 61.5 | 56.5 | 40.0 | 26.5 |
| $10^2$ ng/ml | 48.0 | 43.5 | 29.5 | 16.5 |
| 0 ng/ml | 58.5 | 49.0 | 32.5 | 19.5 |

Cpd. 2 caused an increase in CTL activity, expressed as % lysis, at several concentrations irrespective of the E/T ratio employed or whether or not stimulator cells were present. The one exception was the $10^2$ ng/ml dose when no stimulator cells were present.

Example 28

An MLTC assay was performed to assess the CTL activity and tumour target cell specificity of Cpd. 1. Tumour dormant spleen cells and normal spleen cells of DBA/2 mice were used as effector cells. Irradiated L5178Y cells were used as stimulator cells. $^{51}Cr$-labelled L5178Y cells and FLC-745 cells were used as target cells. The effector/target ratios studied were 100:1, 30:1 and 10:1. The results are shown in Table 21.

TABLE 21

Effect of Cpd. 1 *In Vitro*, as Measured by MLTC Assay, on TDS and Normal Spleen Cells from the DBA/2 Strain of Mice

| | % Lysis | | |
|---|---|---|---|
| | E/T Ratio | | |
| Target cell - L5178Y | 100:1 | 30:1 | 10:1 |
| E + S + D | 82.3 | 69.2 | 34.9 |
| E + S | 55.5 | 39.3 | 11.4 |
| E + D | 52.8 | 29.8 | 9.0 |
| E | 50.9 | 29.2 | 7.2 |
| N + S + D | -9.5 | -9.7 | -10.9 |
| N + S | -14.2 | -10.5 | -11.2 |
| N + D | -12.4 | -11.4 | -10.3 |
| N | -11.2 | -11.0 | -10.9 |
| Target cell - FLC-745 | | | |
| E + S + D | 13.4 | 3.6 | 0.5 |
| E + S | 6.9 | 1.4 | -4.0 |
| E + D | 6.6 | 4.6 | 0.7 |
| E | 11.8 | 6.6 | -1.3 |
| N + S + D | 5.8 | -0.8 | -1.1 |
| N + S | 5.8 | 1.1 | -2.6 |
| N + D | 13.9 | 0.9 | -3.5 |
| N | 1.7 | -1.8 | -2.9 |

E = Effector, Tumour dormant spleen cells; S = Stimulator, Irradiated L5178Y cells; D = Cpd. 1; N = Normal effector cells, Normal spleen cells.

A significant specific enhancement of CTL activity of effector cells in the presence of stimulator cells was noted in the L5178Y target control system when Cpd. 1 was present. This effect was not observed in the FLC-745 target control system nor did effector cells alone yield an enhancement effect. These results demonstrate the enhancement of the activity of stimulated effector cells by Cpd. 1, the activity of the T-cell memory of the system, and the specificity of the stimulated effector cells.

Example 29

The effect of Cpd. 1 on the establishment of the L5178Y tumour dormant state was studied by treating forty DBA/2 mice with Cpd. 1 after the mice had undergone the TDS inducing procedure. Two days after the mice had been challenged with i.p. administration of 50,000 viable L5178Y tumor cells, twenty of these mice received seven consecutive days of i.p. treatment with Cpd. 1 at 100 mg/kg and the remaining twenty mice received buffer only. A partial peritoneal lavage was performed twenty-five days after the last dose of Cpd. 1 and the peritoneal exudate fluid was plated out for determination of tumour cell numbers (tumour cell quantitative assay). Survival time was recovered out to 90 days after challenge with the viable L5178Y tumour cells. The percent mortality was calculated for each group. The results are shown in Tables 22—A and 22—B.

TABLE 22-A

Range Distribution of Tumour Cells in TDS DBA/2 Mice, as Measured by Tumour Cell Quantitative Assay

| No. Tumour Cells | Control Group Frequency | Control Group % of Total | Treated Group Frequency | Treated Group % of Total |
|---|---|---|---|---|
| 0 | 1 | 6 | 4 | 22 |
| <1,000 | 6 | 35 | 6 | 33 |
| 1,000 to 100,000 | 8 | 47 | 7 | 39 |
| >100,000 | 2 | 12 | 1 | 6 |

TABLE 22-B

Mortality Statistics for TDS DBA/2 Mice at 90 days Post-Challenge

| Cpd. 1 Concentration | No. Survivors | % Survival |
|---|---|---|
| 100 mg/kg | 10 | 55.6 |
| 0 mg/kg | 3 | 17.6 |

There was a significant reduction in the numbers of tumour cells recovered from Cpd. 1 treated animals versus the controls. The treated group showed more mice demonstrating lower tumour cell numbers in peritoneal washes. There was a significantly greater survival of these mice treated with Cpd. 1 over untreated controls.

Example 30

The effect of Cpd. 1 on the establishment of L5178Y tumour dormant state was studied by treating forty DBA/2 mice with Cpd. 1 after the mice had undergone the TDS inducing procedure. Two days afte the mice were challenged with i.p. administration of 50,000 viable L5178Y tumour cells, twenty of these mice received seven consecutive days of i.p. treatment with Cpd. 1 at 100 mg/kg and the remaining twenty mice received buffer only. A partial peritoneal lavage was performed twenty-five days after the last dose of Cpd.

1 and the peritoneal exudate fluid was plated out for determination of tumour cell numbers (tumour cell quantitative assay). Survival time was recorded out to 114 days after challenge with the viable L5178Y tumour cells. The percent mortality was calculated for each group. The results are shown in Tables 23—A and 23—B.

TABLE 23-A

Range Distribution of Tumour Cells in TDS DBA/2 Mice, as Measured by Tumour Cell Quantitative Assay

| No. Tumour Cells | Control Group Frequency | Control Group % of Total | Treated Group Frequency | Treated Group % of Total |
|---|---|---|---|---|
| 0 | 3 | 13.0 | 16 | 69.6 |
| <1,000 | 8 | 34.8 | 2 | 8.7 |
| 1,000 to 100,000 | 10 | 43.5 | 4 | 17.4 |
| >100,000 | 2 | 8.7 | 1 | 4.3 |

TABLE 23-B

Mortality Statistics for TDS DBA/2 Mice at 114 Days Post-Challenge

| Cpd 1 Concentration | No. Survivors | % Survival |
|---|---|---|
| 100 mg/kg | 19 | 82.6 |
| 0 mg/kg | 11 | 47.8 |

There was significant reduction in the numbers of tumour cells recovered from Cpd. 1 treated animals versus the controls. The treated group showed more mice demonstrating lower tumour cell numbers in peritoneal washes. There was a significantly greater survival of mice treated with Cpd. 1 over untreated controls.

Example 31

Mice of the $BDF_1$ strain, inoculated with L—1210 leukaemia cells, were treated for seven days with either 25, 50, 100, 200 or 400 mg/kg of Cpd. 1 or just buffer. The National Cancer Institute protocol for screening new anti-cancer agents was followed and T/C ratios were calculated. The results are shown in Table 24.

TABLE 24

Effect of Cpd. 1 on the Treatment of L-1210 Leukaemia as Measured by T/C Ratios, in $BDF_1$ Strain of Mice

| Cpd. 1 Conc. | Median Survival Time (Days) | T/C |
|---|---|---|
| 400 mg/kg | 25 | 1.25 |
| 200 mg/kg | 30 | 1.50 |
| 100 mg/kg | 30 | 1.50 |
| 50 mg/kg | 21 | 1.05 |
| 25 mg/kg | 20 | 1.00 |
| 0 mg/kg | 20 | --- |

The doses of 400, 200 and 100 mg/kg yield T/C values of 1.25, 1.50 and 1.50 respectively, which are considered significant by National Cancer Institute standards of evaluation.

Example 32

Mice of the $BDF_1$ strain, inoculated with L—1210 leukaemia cells, were treated for seven days with either 100 mg/kg of Cpd. 1, 20 mg/kg of 5-fluorouracil (5—FU), a known anti-cancer agent, or just buffer. The National Cancer Institute protocol for screening new anti-cancer agents was followed and T/C ratios are calculated. The results are shown in Table 25.

TABLE 25

Effect of Cpd. 1 and 5-FU on the Treatment of L-1210 Leukaemia, as Measured by T/C Ratios, in $BDF_1$ Strain of Mice

| Drug | Conc. | Median Survival Time | T/C |
|---|---|---|---|
| Cpd. 1 | 100 mg/kg | 28 | 1.40 |
| 5-FU | 20 mg/kg | 30 | 1.50 |
| Buffer | --- | 20 | --- |

The 100 mg/kg Cpd. 1 treatment dose yielded a T/C value of 1.40 compared to 1.50 for the 5—FU positive control. This data substantiated the positive findings of Example 31 and also compared the anti-cancer results observed after use of Cpd. 1 to the results observed after use of an accepted positive control drug, 5—FU.

**Claims**

1. Compounds of general formula (I):

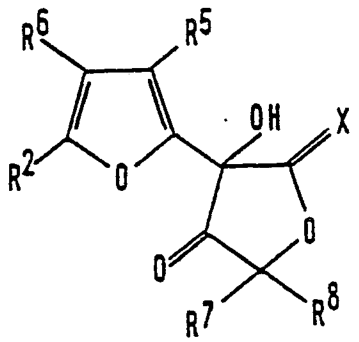

(I)

in which:

$R^2$ represents a hydrogen atom or a $C_1$—$C_6$ alkyl group;

$R^5$ and $R^6$ are the same or different and each represents a hydrogen atom or a $C_1$—$C_6$ alkyl group;

$R^7$ and $R^8$ are the same or different and each represents a hydrogen atom or a $C_1$—$C_6$ alkyl group, or $R^7$ represents a group of formula:

$$R^9(H)C(OR^{10})—$$

in which:

$R^9$ represents a group of formula

$$—(CH_2)_n—OR^{11};$$

$n$ is an integer from 1 to 3; and $R^{10}$ and $R^{11}$ are the same or different and each represents a hydrogen atom, a $C_1$—$C_6$ alkyl group, a phenyl group or a hydroxy-substituted $C_1$—$C_6$ alkyl group; and

X represents an oxygen or sulphur atom or the group = NH; and

where $R^7$ contains a hydroxy group, hemiketels thereof; and

adducts of said compunds of general formula (I) or said hemiketals with an anhydride or amide of succinic acid.

2. Compounds as claimed in claim 1, in which: $R^2$ represents a $C_1$—$C_3$ alkyl group; $R^5$ and $R^6$ are the same or different and each represents a hydrogen atom or a $C_1$—$C_3$ alkyl group; X represents an oxygen atom; $R^8$ represents a hydrogen atom or a $C_1$—$C_3$ alkyl group; and $R^7$ represents said group of formula:

$$R^9(H)C(OR^{10})—$$

in which: $R^9$ represents —$(CH_2)_n$—$OR^{11}$ *n* is an ingeger from 1 to 3; $R^{10}$ and $R^{11}$ are the same or different and each represents a hydrogen atom or a $C_1$—$C_3$ alkyl group.

3. Compounds as claimed in claim 2, wherein: $R^2$ represents a methyl group; and $R^5$ and $R^6$ each represent hydrogen atoms.

4. Compounds as claimed in claim 2, in which: $R^9$ represents —$CH_2OH$; and $R^{10}$ represents a hydrogen atom.

5. Compounds as claimed in any one of claims 1 to 4, in which $R^7$ forms a hemiketal ring closure at carbon 3 of the butyrolactone, with protonation of the carbonyl group at carbon 3.

6. Compounds as claimed in any one of claims 1 to 5 in which said anhydride or amide is succinic anhydride, succinimide or *N*-methylsuccinimide.

7. Compounds as claimed in any one of claims 1 to 6, in which said anhydride or amide is present in an amount of at least 0.5 mole per mole of said compound (I).

8. Compounds as claimed in claim 7, wherein said amount is 0.5 mole.

9. A pharmaceutical composition comprising a compound as claimed in any one of claims 1 to 8 in admixture with a pharmaceutically acceptable carrier or diluent.

10. A method of preparing a compound as claimed in any one of claims 1 to 8, which method comprises:

(a) reacting a compound of the general formula (II):

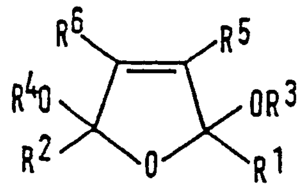

(II)

wherein:

$R^1$ and $R^2$ are the same or different and each represents a hydrogen atom or a $C_1$—$C_6$ alkyl group, provided that at least one is hydrogen;

$R^3$ and $R^4$ are the same or different and each represents a $C_1$—$C_6$ alkyl group or an aryl group; and

$R^5$ and $R^6$ are as defined in claim 1 with a compound of the general formula (III):

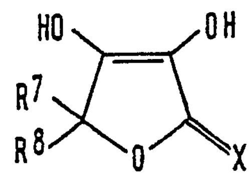

(III)

in which X, $R^7$ and $R^8$ are as defined in claim 1;

(b) treating the product with an anhydride or an amide of succinic acid;

(c) separating the product as an adduct with said anhydride or amide from the reaction mixture; and

(d) if desired, dissociating said product into said compound of general formula (I) or hemiketal thereof and said anhydride or amide.

11. A method as claimed in claim 10, wherein there is employed as said compound of general formula (II) a compound n which: $R^1$ represents a hydrogen atom or a $C_1$—$C_3$ alkyl group; $R^2$ represents a $C_1$—$C_3$ alkyl group; $R^3$ and $R^4$ are the same or different and each represents a $C_1$—$C_3$ alkyl group; and $R^5$ and $R^6$ are the same or different and each represents a hydrogen atom or a $C_1$—$C_3$ alkyl group.

12. A method as claimed in claim 11, wherein: $R^2$ represents a methyl group; and $R^5$ and $R^6$ each represent hydrogen atoms.

13. A method as claimed in claim 12, wherein said compound of general formula (II) is 2-methyl-2,5-dimethoxy-2,5-dihydrofuran.

14. A method as claimed in any one of claims 10 to 13, wherein said compound of general formula (III) is a compound in which: X represents an oxygen atom; $R^8$ represents a hydrogen atom or a $C_1$—$C_3$ alkyl group; and $R^7$ represents said group of formula:

$$R^9(H)C(OR^{10})—$$

in which: $R^9$ represents $—(CH_2)_nOR^{11}$ *n* is an integer from 1 to 3; $R^{10}$ and $R^{11}$ are the same or different and each represents a hydrogen atom or a $C_1—C_3$ alkyl group.

15. A method as claimed in claim 14, in which: $R^9$ represents $—CH_2OH$; and $R^{10}$ represents a hydrogen atom.

16. A method as claimed in claim 15, in which said compound of general formula (III) is *L*-ascorbic acid.

17. A method as claimed in any one of claims 10 to 16, in which $R^7$ forms a hemiketal ring closure at carbon 3 of the butyrolactone, with protonation of the carbonyl group at carbon 3.

18. A method as claimed in any one of claims 10 to 17, in which the molar ratio of said compound (II) to said compound (III) is from 2:1 to 1:2.

19. A method as claimed in claim 18, in which said molar ratio is 1:1.

20. A method as claimed in any one of claims 10 to 19 in which said anhydride or amide is succinic anhydride, succinimide or *N*-methylsuccinimide.

21. A method as claimed in any one of claims 10 to 20, in which said anhydride or amide is employed in an amount of at least 0.5 mole per mole of said compound (I).

22. A method as claimed in claim 21, wherein said amount is 0.5 mole.

**Patentansprüche**

1. Verbindungen der allgemeinen Formel (I):

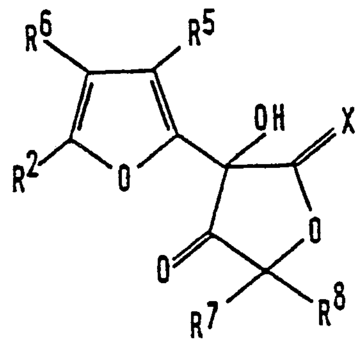

(I)

in der:

$R^2$ ein Wasserstoffatom oder eine $C_1—C_6$-Alkylgruppe repräsentiert;

$R^5$ und $R^6$ gleich oder verschieden sind und jeder dieser Reste ein Wasserstoffatom oder eine $C_1—C_6$-Alkylgruppe repräsentieren;

$R^7$ und $R^8$ gleich oder verschieden sind und jeweils ein Wasserstoffatom oder eine $C_1—C_6$-Alkylgruppe oder $R^7$ eine Gruppe der Formel:

$R^9(H)C(OR^{10})—$ repräsentieren,

wobei

$R^9$ eine Gruppe der Formel

$—(CH_2)_n—OR^{11}$ repräsentiert,

wobei

n eine ganze Zahl von 1 bis 3 ist und wobei $R^{10}$ und $R^{11}$ gleich oder verschieden sind und jeweils ein Wasserstoffatom, eine $C_1—C_6$-Alkylgruppe, eine Phenylgrupe oder eine hydroxysubstituierte $C_1—C_6$-Alkylgruppe repräsentieren;

und wobei

X ein Sauerstoff- oder ein Schwefelatom ode die Gruppe = NH repräsentiert;

und wobei

$R^7$ eine Hydroxygruppe oder ein Halbketal davon enthält;

und Addukte der Verbindung der allgemeinen Formel (I) oder der Halbketale mit einem Anhydrid oder einem Amid der Bernsteinsäure.

2. Verbindungen gemäß Anspruch 1, wobei $R^2$ eine $C_1—C_3$-Alkylgruppe repräsentiert; $R^5$ und $R^6$ gleich oder verschieden sind und jeweils ein Wasserstoffatom oder eine $C_1—C_3$-Alkylgruppe repräsentieren; X ein Sauerstoffatom repräsentiert; $R^8$ ein Wasserstoffatom oder eine $C_1—C_3$-Alkylgruppe repräsentiert; und $R^7$ eine Gruppe der Formel $R^9(H)C(OR^{10})—$ repräsentiert, bei der $R^9$ einen $—(CH_2)_nOR^{11}$-Rest repräsentiert, wobei n eine ganze Zahl von 1 bis 3 ist und $R^{10}$ und $R^{11}$ gleich oder verschieden sind und jeweils ein Wasserstoffatom oder eine $C_1—C_3$-Alkylgruppe repräsentieren.

3. Verbindungen gemäß Anspruch 2, wobei $R^2$ eine Methylgruppe und $R^5$ und $R^6$ jeweils ein Wasserstoffatom repräsentieren.

4. Verbindungen gemäß Anspruch 2, wobei $R^9$ eine $-CH_2OH$-Gruppe und $R^{10}$ ein Wasserstoffatom repräsentieren.

5. Verbindungen gemäß einem oder mehreren der Ansprüche 1 bis 4, wobei $R^7$ einen Halbketal-Ringschluß am C-Atom 3 des Butyrolactonrests unter Protonierung der Carbonylgruppe am C-Atom 3 bildet.

6. Verbindungen gemäß einem oder mehreren der Ansprüche 1 bis 5, wobei das Anhydrid oder das Amid Succinanhydrid, Succinimid oder N-Methylsuccinimid ist.

7. Verbindungen gemäß einem oder mehreren der Ansprüche 1 bis 6, wobei das Anhydrid oder das Amid in einer Menge von mindestens 0,5 Mol pro Mol der Verbindung (I) vorhanden ist.

8. Verbindungen gemäß Anspruch 7, wobei das Anhydrid oder das Amid in einer Menge von 0,5 Mol pro Mol der Verbindung (I) vorhanden ist.

9. Pharmazeutische Zusammensetzung, die eine Verbindung gemäß einem oder mehreren der Ansprüche 1 bis 8 in einer Mischung mit einem pharmazeutisch akzeptablen Träger oder Verdünnungsmittel umfaßt.

10. Verfahren zur Herstellung einer Verbindung gemäß einem oder mehreren der Ansprüche 1 bis 8, wobei das Verfahren folgende Schritte umfaßt:

a) Reaktion einer Verbindung der allgemeinen Formel (II):

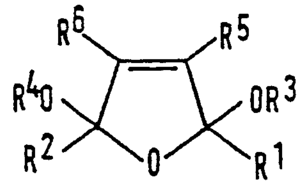

(II)

wobei

$R^1$ und $R^2$ gleich oder verschieden sind und jeweils ein Wasserstoffatom oder eine $C_1$—$C_6$-Alkylgruppe repräsentieren, wobei mindestens einer der Reste ein Wasserstoffatom ist;

$R^3$ und $R^4$ gleich oder verschieden sind und jeweils eine $C_1$—$C_6$-Alkylgruppe oder eine Arylgruppe repräsentieren; und wobei

$R^5$ und $R^6$ wie in Anspruch 1 definiert sind, mit einer Verbindung der allgemeinen Formel (III)

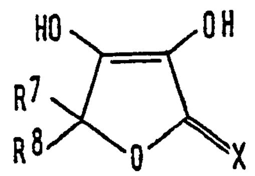

(III)

wobei X, $R^7$ und $R^8$ wie in Anspruch 1 definiert sind;

b) Behandlung des Produkts mit einem Anhydrid oder einem Amid der Bernsteinsäure;

c) Abtrennen des Produkts als Addukt mit dem Anhydrid oder Amid aus der Reaktionsmischung; und

d) gegebenenfalls Dissoziieren des Produkts in die Verbindung der allgemeinen Formel (I) oder ein Halbketal hiervon und das Anhydrid oder das Amid.

11. Verfahren gemäß Anspruch 10, wobei als Verbindung der allgemeinen Formel (II) eine Verbindung verwendet wird, in der $R^1$ eine Wasserstoffatom oder eine $C_1$—$C_3$-Alkylgruppe repräsentiert; $R^2$ eine $C_1$—$C_3$-Alkylgruppe repräsentiert; $R^3$ und $R^4$ gleich oder verschieden sind und jeweils eine $C_1$—$C_3$-Alkylgruppe repräsentieren; und $R^5$ und $R^6$ gleich oder verschieden sind und jeweils ein Wasserstoffatom oder eine $C_1$—$C_3$-Alkylgruppe repräsentieren.

12. Verfahren gemäß Anspruch 11, wobei $R^2$ eine Methylgruppe und $R^5$ und $R^6$ jeweils ein Wasserstoffatom repräsentieren.

13. Verfahren gemäß Anspruch 12, wobei die Verbindung der allgemeinen Formel (II) 2-Methyl-2,5-dimethoxy-2,5-dihydrofuran ist.

14. Verfahren nach einem oder mehreren der Ansprüche 10 bis 13, wobei die Verbindung der allgemeinen Formel (III) eine Verbindung ist, in der: X ein Sauerstoffatom repräsentiert; $R^8$ ein Wasserstoffatom oder eine $C_1$—$C_3$-Alkylgruppe repräsentiert; und $R^7$ eine Gruppe der Formel $R^9(H)C(OR^{10})$— repräsentiert, in der: $R^9$ einen $-(CH_2)_nOR^{11}$-Rest repräsentiert, wobei n eine ganze Zahl von 1 bis 3 ist und wobei $R^{10}$ und $R^{11}$ gleich oder verschieden sind und jeweils ein Wasserstoffatom oder eine $C_1$—$C_3$-Alkylgruppe repräsentieren.

15. Verfahren gemäß Anspruch 14, wobei $R^9$ einen $-CH_2OH$-Rest und $R^{10}$ ein Wasserstoffatom repräsentieren.

16. Verfahren gemäß Anspruch 15, wobei die Verbindung der allgemeinen Formel (III) L-Ascorbinsäure ist.

17. Verfahren gemäß einem oder mehreren der Ansprüche 10 bis 16, wobei $R^7$ einen Halbketal-Ringschluß am C-Atom 3 des Butyrolactons unter Protonierung der Carbonylgruppe am C-Atom 3 bildet.

18. Verfahren gemäß einem oder mehreren der Ansprüche 10 bis 17, wobei das molare Verhältnis der Verbindung (II) zu der Verbindung (III) 2:1 bis 1:2 beträgt.

19. Verfahren gemäß Anspruch 18, wobei das molare Verhältnis 1:1 beträgt.

20. Verfahren gemäß einem oder mehreren der Ansprüche 10 bis 19, wobei das Anhydrid oder Amid Succinanhydrid, Succinimid oder N-Methylsuccinimid ist.

21. Verfahren gemäß einem oder mehreren der Ansprüche 10 bis 20, worin das Anhydrid oder Amid in einer Menge von mindestens 0,5 Mol pro Mol der Verbindung (I) verwendet wird.

22. Verfahren gemäß Anspruch 21, worin die verwendete Menge 0,5 Mol pro Mol der Verbindung (I) beträgt.

**Revendications**

1. Composés de formule générale (I):

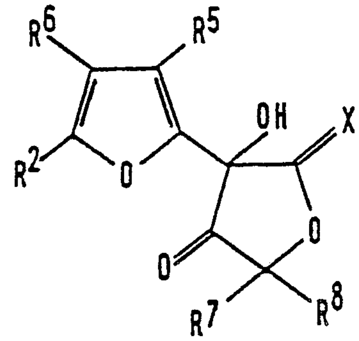

(I)

dans lesquels:

$R^2$ représente un atome d'hydrogène ou un groupe alkyle $C_1$ à $C_6$,

$R^5$ et $R^6$ sont identiques ou différents et chacun représente un atome d'hydrogène ou un groupe alkyle $C_1$ à $C_6$,

$R^7$ et $R^8$ sont identiques ou différents et chacun représente un atome d'hydrogène ou un groupe alkyle $C_1$ à $C_6$, ou $R^7$ représente un groupe de formule:

$$R^9(H)C(OR^{10})—$$

dans lequel: $R^9$ représente un groupe de formule:

$$—(CH_2)_nOR^{11},$$

n est un entier de 1 à 3, et $R^{10}$ et $R^{11}$ sont identiques ou différents et chacun représente un atome d'hydrogéne, un groupe alkyle $C_1$ à $C_6$, un groupe phényl ou un groupe alkyle hydroxy-substitué $C_1$ à $C_6$; et

X représente un atome d'oxygène ou de soufre, ou le groupe = NH, et

où $R^7$ contient un groupe hydroxy, des hémiacétals de celui-ci, et

des produits d'addition dudit composé de formule générale (I) ou lesdits hemi-acétals avec un anhydride ou un amide de l'acide succinique.

2. Composés selon la revendication 1, caractérisés en ce que: $R^2$ représente un groupe alkyle $C_1$ à $C_3$; $R^5$ et $R^6$ sont identiques ou différents et chacun représente un atome d'hydrogène ou un groupe alkyle $C_1$ à $C_3$, X représente un atome d'oxygène, $R^8$ représente un atome d'hydrogène ou un groupe alkyle $C_1$ à $C_3$; et $R^7$ représente ledit groupe de formule: $R^9(H)C(OR^{10})—$ dans lequel $R^9$ représente $—(CH_2)_nOR^{11}$ n est un entier de 1 à 3, $R^{10}$ et $R^{11}$ sont identiques ou différents et chacun représente un atome d'hydrogène ou un groupe alkyle de $C_1$ à $C_3$.

3. Composés selon la revendication 2, caractérisés en ce que $R^2$ représente un groupe méthyle, et $R^5$ et $R^6$ représentent chacun des atomes d'hydrogène.

4. Composés selon la revendication 2, caractérisés en ce que $R^9$ représente $—CH_2OH$; et $R^{10}$ représente un atome d'hydrogène.

5. Composés selon l'une quelconque des revendications 1 à 4, caractérisés en ce que $R^7$ forme un anneau fermé hémi-acétal sur le carbone 3 du butyrolactone, avec protonation du groupe carbonyle au niveau du carbone 3.

6. Composés selon l'une quelconque des revendications 1 à 5, caractérisés en ce que ledit anhydride ou amide est l'anhydride succinique, le succinimide ou le N-méthylsuccinimide.

7. Composés selon l'une quelconque des revendications 1 à 6, caractérisés en ce que ledit anhydride ou amide est présent selon une quantité d'au moins 0,5 mole par mole dudit composé (I).

8. Composés selon la revendication 7, caractérisés en ce que ladite quantité est de 0,5 mole.

9. Composition pharmaceutique caractérisée en ce qu'elle comprend un composé selon l'une quelconque des revendications 1 à 8 mélangé à un porteur ou un diluant acceptable pharmaceutiquement.

10. Procédé de préparation d'un composé selon l'une quelconque des revendications 1 à 8, procédé caractérisé en ce qu'il comprend les étapes consistant à:

a) faire réagir un composé de formule générale (II):

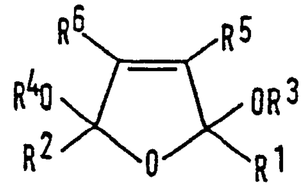

(II)

dans lequel

$R^1$ et $R^2$ sont identiques ou différents et chacun représente un atome d'hydrogène ou un groupe alkyle $C_1$ à $C_6$, pourvu que l'un au moins soit l'hydrogène,

$R^3$ et $R^4$ sont identiques ou différents et chacun représente un groupe aryle ou un groupe alkyle $C_1$ à $C_6$; et

$R^5$ et $R^6$ sont tels que définis dans la revendication 1 avec un composé de formule générale (III):

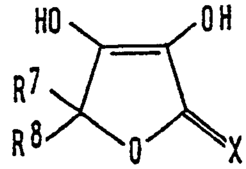

(III)

dans laquel X, $R^7$ et $R^8$ sont tels que définis dans la revendication 1;

b) traiter le produit avec un anhydride ou un amide d'acide succinique;

c) séparer le produit comme un produit d'addition avec ledit anhydride ou amide du mélange réactionnel; et

d) si nécessaire, dissocier ledit produit en ledit composé de formule générale (I) ou un hemi-acétal de celui-ci et l'anhydride ou l'amide.

11. Procédé selon la revendication 10, caractérisé en ce qu'on emploie comme composé de formule générale (II) un composé dans lequel: $R^1$ représente un atome d'hydrogène ou un groupe alkyle $C_1$ à $C_3$; $R^2$ représente un groupe alkyle $C_1$ à $C_3$, $R^3$ et $R^4$ sont identiques ou différents et chacun représente un groupe alkyle $C_1$ à $C_3$, et $R^5$ et $R^6$ sont identiques ou différents et chacun représente un atome d'hydrogène ou un groupe alkyle $C_1$ à $C_3$.

12. Procédé selon la revendication 11, caractérisé en ce que: $R^2$ représente un groupe méthyle; et $R^5$ et $R^6$ représentent chacun des atomes d'hydrogène.

13. Procédé selon la revendication 12, caractérisé en ce que ledit composé de formule générale (II) est le méthyl-2-diméthoxy-2,5-dihydro-2,5 furanne.

14. Procédé selon l'une quelconque des revendications 10 à 13, caractérisé en ce que ledit composé de formule générale (III) est un composé dans lequel X représente un atome d'oxygène, $R^8$ représente un atome d'hydrogène ou un groupe alkyle $C_1$ à $C_3$; et $R^7$ représente ledit groupe de formule: $R^9(H)C(OR^{10})$—, dans lequel $R^9$ représente $-(CH_2)_nOR^{11}$, n est un entier de 1 à 3; $R^{10}$ et $R^{11}$ sont identiques ou différents et chacun représente un atome d'hydrogène ou un groupe alkyle $C_1$ à $C_3$.

15. Procédé selon la revendication 14, caractérisé en ce que: $R^9$ représente $-CH_2OH$, et $R_{10}$ représente un atome d'hydrogène.

16. Procédé selon la revendication 15, caractérisé en ce que ledit composé de formule générale (III) est l'acide L-ascorbique.

17. Procédé selon l'une quelconque des revendications 10 à 16, caractérisés en ce que $R^7$ forme un anneau fermé hemi-acétal sur le carbone 3 du butyrolactone avec protonation du groupe carbonyle sur le carbone 3.

18. Procédé selon l'une quelconque des revendications 10 à 17, caractérisés en ce que le rapport molaire dudit composé (II) audit composé (III) varie de 2:1 à 1:2.

19. Procédé selon la revendication 18, caractérisé en ce que ledit rapport molaire est de 1:1.

20. Procédé selon l'une quelconque des revendications 10 à 19, caractérisé en ce que ledit anhydride ou amide est l'anhydride succinique, le succinimide ou le N-méthylsuccinimide.

21. Procédé selon l'une quelconque des revendications 10 à 20, caractérisés en ce que ledit anhydride ou amide est employé selon une quantité d'au moins 0,5 mole par mole dudit composé (I).

22. Procédé selon la revendication 21, caractérisé en ce que ladite quantité est de 0,5 mole.